(19) Europäisches Patentamt
European Patent Office
Office européen des brevets

(11) Veröffentlichungsnummer : **0 309 864 B1**

(12) # EUROPÄISCHE PATENTSCHRIFT

(45) Veröffentlichungstag der Patentschrift : **22.05.91 Patentblatt 91/21**

(51) Int. Cl.$^5$ : **C07C 59/68**, C07C 69/736, C07C 51/00, C07C 67/00, A01N 39/02, C07C 43/295

(21) Anmeldenummer : **88115372.0**

(22) Anmeldetag : **20.09.88**

(54) **Alpha-(5-Aryloxy-naphthalin-1-yl-oxy)-propionsäure-Derivate.**

(30) Priorität : **30.09.87 DE 3733067**

(43) Veröffentlichungstag der Anmeldung : **05.04.89 Patentblatt 89/14**

(45) Bekanntmachung des Hinweises auf die Patenterteilung : **22.05.91 Patentblatt 91/21**

(84) Benannte Vertragsstaaten : **BE CH DE FR GB IT LI NL**

(56) Entgegenhaltungen :
**EP-A- 0 179 015**
**CHEMICAL ABSTRACTS, Band 107, Nr. 12, 21. September 1987, Columbus, Ohio, USA; SATAKE, HISAMI et al.: "Thermal recording materials", Seite 655, Spalte 2, Zusammenfassung Nr. 106 424j.**

(73) Patentinhaber : **BAYER AG**
**W-5090 Leverkusen 1 Bayerwerk (DE)**

(72) Erfinder : **Haug, Michael, Dr.**
**Fahner Weg 5**
**W-5060 Bergisch Gladbach 2 (DE)**
Erfinder : **Santel, Hans-Joachim, Dr.**
**Grünstrasse 9a**
**W-5090 Leverkusen 1 (DE)**
Erfinder : **Schmidt, Robert R., Dr.**
**Im Waldwinkel 110**
**W-5060 Bergisch Gladbach 2 (DE)**
Erfinder : **Strang, Harry, Dr.**
**Unterdorfstrasse 6A**
**W-4000 Düsseldorf 31 (DE)**

**EP 0 309 864 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue α-(5-Aryloxy-naphthalin-1-yl-oxy)-propionsäure-Derivate, Verfahren und neue Zwischenprodukte zu ihrer Herstellung sowie ihre Verwendung als Herbizide.

Es ist bereits bekannt, daß bestimmte 7-Aryloxy-naphthalin-2-yl-oxy-carbonsäure-Derivate, wie z.B. α-(7-(2-Chlor-4-trifluormethyl-phenoxy)-naphthalin-2-yl-oxy)-propionsäure-ethylester, herbizid wirksam sind (vgl. EP-A 179 015). Die Wirkung dieser bekannten Verbindungen gegen Unkräuter sowie ihre Kulturplanzen-Verträglichkeit sind jedoch nicht immer zufriedenstellend.

Es wurden nun neue α-(5-Aryloxy-naphthalin-1-yl-oxy)-propionsäure-Derivate der allgemeinen Formel (I)

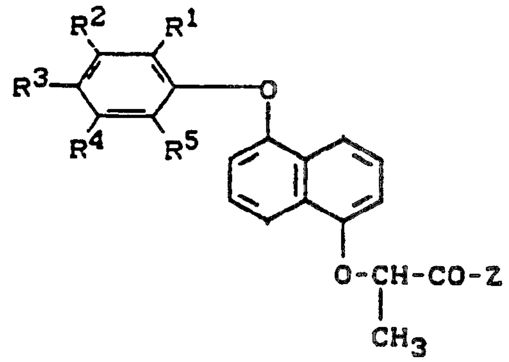

(I)

in welcher

$R^1$ für Wasserstoff, Halogen, Cyano oder Trifluormethyl steht,
$R^2$ für Wasserstoff oder Halogen steht,
$R^3$ für Halogen, Cyano, Trifluormethyl oder Trifluormethoxy steht,
$R^4$ für Wasserstoff oder Halogen steht,
$R^5$ für Wasserstoff oder Halogen steht und
Z für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung $-O-R^6$ steht, worin
$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl, Alkylidenamino oder für ein Ammonium-, Alkylammonium-, Alkalimetall- oder Erdalkalimetall-äquivalent steht oder für die Gruppierung

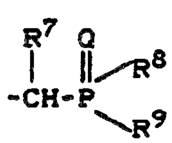

steht, worin
$R^7$ für Wasserstoff, Alkyl, Aryl, Furyl, Thienyl oder Pyridyl steht,
$R^8$ für Alkyl oder Alkoxy steht,
$R^9$ für Alkoxy steht und
Q für Sauerstoff oder Schwefel steht,

oder

$R^6$ für die Gruppierung $-(CH_2)_n-R^{10}$ steht, worin
$R^{10}$ für einen gegebenenalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht, gefunden.

Die Verbindungen der Formel (I) enthalten mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Weiter wurde gefunden, daß man die neuen α-(5-Aryloxy-naphthalin-1-yl-oxy)-propionsäure-Derivate der Formel (I) erhält, wenn man

(a) 5-Aryloxy-1-naphthole der allgemeinen Formel (II)

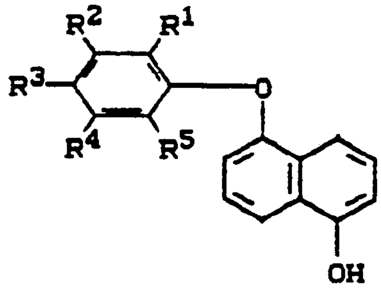

(II)

in welcher

$R^1$, $R^2$, $R^3$, $R^4$ und $R^5$ die oben angegebenen Bedeutungen haben, mit Propionsäure-Derivaten der allgemeinen Formel (III)

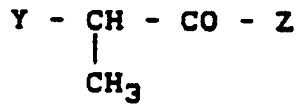

(III)

in welcher

Z die oben angegebene Bedeutung hat und

Y für eine nucleophile Abgangsgruppe steht, gegebenenfalls in Gegenwart eines Säureakzeptors und gegebenenfalls in Gegenwart eines Verdünnungsmittels umsetzt, oder

(b) Halogen-benzol-Derivate der allgemeinen Formel (IV)

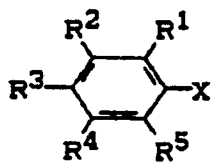

(IV)

in welcher

$R^1$, $R^2$, $R^3$, $R^4$ und $R^5$ die oben angegebenen Bedeutungen haben und

X für Halogen steht, mit α-(5-Hydroxy-naphthalin-1-yl-oxy)-propionsäure-Derivaten der allgemeinen Formel (V)

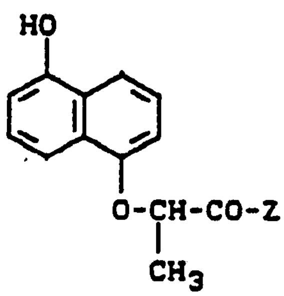

(V)

in welcher
Z die oben angegebene Bedeutung hat, gegebenenfalls in Gegenwart eines Säureakzeptors und gegebenenfalls in Gegenwart eines Verdünnungsmittels umsetzt, oder
(c) für den Fall, daß Z für Hydroxy steht und die Reste $R^1$ bis $R^5$ die oben angegebenen Bedeutungen haben, wenn man Verbindungen der allgemeinen Formel (I), in welcher Z für Methoxy oder Ethoxy steht und die Reste $R^1$ bis $R^5$ die oben angegebenen Bedeutungen haben,
mit einem Alkalihydroxid in Gegenwart von Wasser und gegebenenfalls in Gegenwart eines organischen Lösungsmittels umsetzt und dann – gegebenenfalls nach Einengen – mit einer Mineralsäure ansäuert, oder
(d) für den Fall, daß Z für Halogen steht und die Reste $R^1$ bis $R^5$ die oben angegebenen Bedeutungen haben, wenn man Verbindungen der allgemeinen Formel (I), in welcher Z für Hydroxy steht und die Reste $R^1$ bis $R^5$ die oben angegebenen Bedeutungen haben,
mit einem Halogenierungsmittel gegebenenfalls in Gegenwart eines Katalysators und gegebenenfalls in Gegenwart eines Verdünnungsmittels umsetzt, oder
(e) für den Fall, daß Z mit Ausnahme von Halogen die oben angegebene Bedeutung hat und die Reste $R^1$ bis $R^5$ die oben angegebenen Bedeutungen haben, wenn man Verbindungen der allgemeinen Formel (I), in welcher 7 für Halogen steht und die Reste $R^1$ bis $R^5$ die oben angegebenen Bedeutungen haben,
mit Verbindungen der allgemeinen Formel (VI)

$$H-Z \quad (VI)$$

in welcher
Z mit Ausnahme von Halogen die oben angegebene Bedeutung hat,
gegebenenfalls in Gegenwart eines Säureakzeptors und gegebenenfalls in Gegenwart eines Verdünnungsmittels umsetzt, oder
(f) für den Fall, daß Z für die Gruppierung $-O-R^6$ steht, worin $R^6$ mit Ausnahme von Ammonium, Alkylammonium, Alkalimetall und Erdalkalimetall die oben angegebene Bedeutung hat und die Reste $R^1$ bis $R^5$ die oben angegebenen Bedeutungen haben, wenn man Verbindungen der allgemeinen Formel (I), in welcher Z für Hydroxy steht und die Reste $R^1$ bis $R^5$ die oben angegebenen Bedeutungen haben,
mit Verbindungen der allgemeinen Formel (VII)

$$X^1-R^6 \quad (VII)$$

in welcher
$R^6$ mit Ausnahme von Ammonium, Alkylammonium, Alkalimetall und Erdalkalimetall die oben angegebenen Bedeutungen hat und
$X^1$ für Halogen steht,
gegebenenfalls in Gegenwart eines Säureakzeptors und gegebenenfalls in Gegenwart eines Verdünnungsmittels umsetzt.

Schließlich wurde gefunden, daß die neuen α-(5-Aryloxy-naphthalin-1-yl-oxy)-propionsäure-Derivate der allgemeingen Formel (I) starke herbizide Eigenschaften aufweisen.

Überraschenderweise zeigen die erfindungsgemäßen α-(5-Aryloxy-naphthalin-1-yl-oxy)-propionsäure-Derivate der Formel (I) bei guter Verträglichkeit in wichtigen Kulturpflanzen gegen bestimmte Problemunkräuter, wie insbesondere Hirsearten, wesentlich stärkere Wirkung als α-(7-(2-Chlor-4-trifluormethyl-phenoxy)-naphthalin-2-yl-oxy)-propionsäure-ethylester, welcher ein strukturell ähnlicher vorbekannter Wirkstoff gleicher Wirkungsrichtung ist.

Die Erfindung betrifft vorzugsweise Verbindungen der Formel (I), in welcher
$R^1$ für Wasserstoff, Fluor, Chlor, Brom, Cyano oder Trifluormethyl steht,
$R^2$ für Wasserstoff, Fluor oder Chlor steht,
$R^3$ für Fluor, Chlor, Brom, Cyano, Trifluormethyl oder Trifluormethoxy steht,
$R^4$ für Wasserstoff, Fluor oder Chlor steht,
$R^5$ für Wasserstoff, Fluor, Chlor oder Brom steht und
Z für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung $-O-R^6$ steht, worin

$R^6$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium- oder Calciumäquivalent steht, oder für die Gruppierung

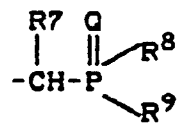

steht, worin

$R^7$ für Wasserstoff, $C_1$-$C_4$-Alkyl, Phenyl, Furyl, Thienyl oder Pyridyl steht,

$R^8$ für $C_1$-$C_4$-Alkyl oder $C_1$-$C_4$-Alkoxy steht,

$R^9$ für $C_1$-$C_4$-Alkoxy steht und

Q für Sauerstoff oder Schwefel steht,

oder

$R^6$ für die Gruppierung $-(CH_2)_n-R^{10}$ steht, worin

n für die Zahlen 0, 1 oder 2 steht und

$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

Die Erfindung betrifft insbesondere Verbindungen der Formel (I), in welcher

$R^1$ für Wasserstoff, Cyano, Fluor oder Chlor steht,

$R^2$ für Wasserstoff, Fluor oder Chlor steht,

$R^3$ für Trifluormethyl steht,

$R^4$ für Wasserstoff, Fluor oder Chlor steht,

$R^5$ für Wasserstoff, Fluor oder Chlor steht und

Z für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl oder für ein Ammonium-, $C_1$-$C_3$-Alkylammonium-, Natrium-, oder Kalium-äquivalent steht, oder für die Gruppierung

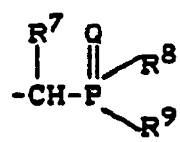

steht, worin

$R^7$ für Wasserstoff, Methyl, Phenyl, Furyl, Thienyl oder Pyridyl steht,

$R^8$ für Methoxy oder Ethoxy steht,

$R^9$ für Methoxy oder Ethoxy steht und

Q für Sauerstoff oder Schwefel steht

oder

$R^6$ für die Gruppierung $(-CH_2-)_n-R^{10}$ steht, worin

n für die Zahlen 0, 1 oder 2 steht und

$R^{10}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht.

Die R-Isomeren der insbesondere bevorzugten Verbindungen der Formel (I) werden ganz besonders bevorzugt.

Verwendet man für das erfindungsgemäße Verfahren (a) beispielsweise 5-(4-Trifluormethyl-phenoxy)-1-naphthol und α-Brom-propionsäure-ethylester als Ausgangsstoffe, so kann der Reaktionsablauf durch das folgende Formelschema wiedergegeben werden :

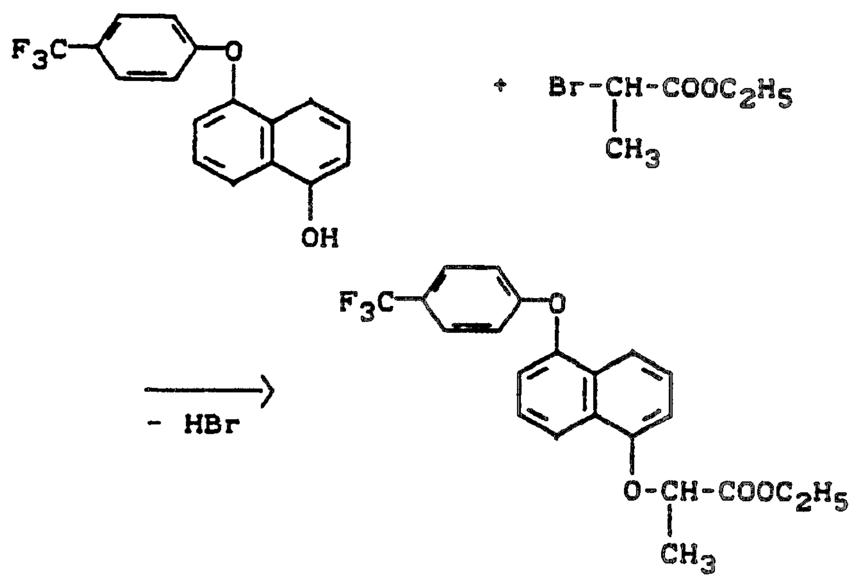


Verwendet man für das erfindungsgemäße Verfahren (b) beispielsweise 3,4,5-Trichlor-benzotrifluorid und α-(5-Hydroxy-naphthalin-1-yl-oxy)-propionsäure-propylester als Ausgangsstoffe, so kann der Reaktionsablauf durch das folgende Formelschema wiedergegeben werden :

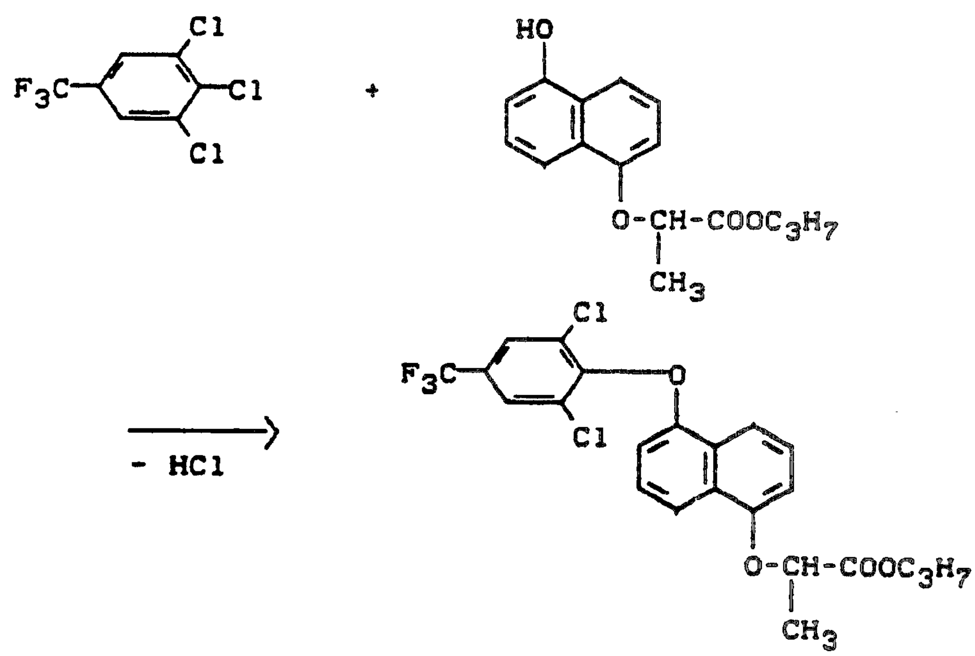


Verwendet man für das erfindungsgemäße Verfahren (c) beispielsweise α-(5-(2-Chlor-4-trifluormethyl-phenoxy)-naphthalin-1-yl-oxy)-propionsäure-methylester und Natronlauge als Ausgangsstoffe, so kann der Reaktionsablauf durch das folgende Formelschema wiedergegeben werden :

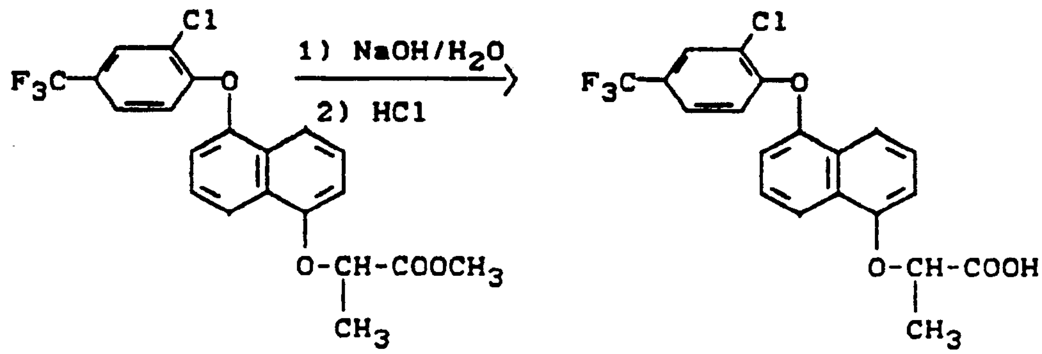


Verwendet man für das erfindungsgemäße Verfahren (d) beispielsweise α-(5-(2,6-Dichlor-3-fluor-4-trifluormethyl-phenoxy)-naphthalin-1-yl-oxy)-propionsäure und Thionylchlorid als Ausgangsstoffe, so kann der Reaktionsablauf durch das folgende Formelschema wiedergegeben werden :

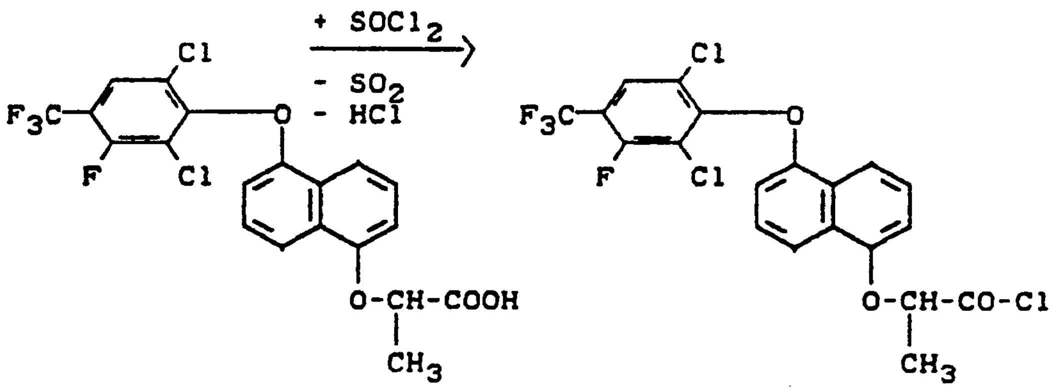


Verwendet man für das erfindungsgemäße Verfahren (e) beispielsweise α-(5-(2-Chlor-6-fluor-4-trifluormethylphenoxy)-naphthalin-1-yl-oxy)-propionsäure-chlorid und Butanol als Ausgangsstoffe, so kann der Reaktionsablauf durch das folgende Formelschema wiedergegeben werden :

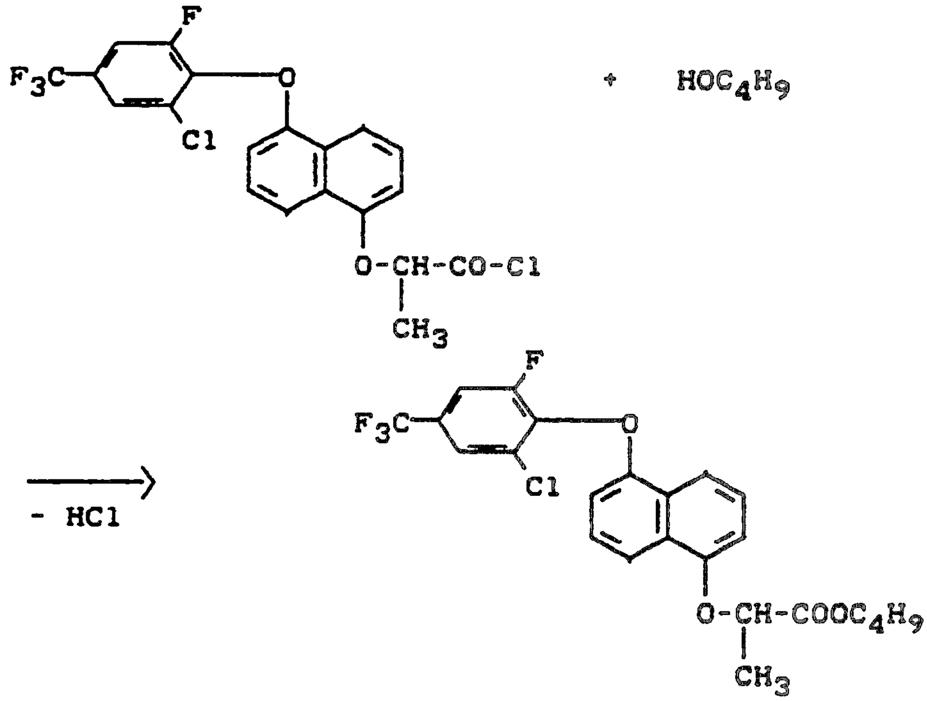


Verwendet man für das erfindungsgemäße Verfahren (f) beispielsweise α-(5-(2,3,6-Trichlor-4-trifluormethylphenoxy)-naphthalin-1-yl-oxy)-propionsäure und Trimethylsilylmethylchlorid als Ausgangsstoffe, so kann der Reaktionsablauf durch das folgende Formelschema wiedergegeben werden :

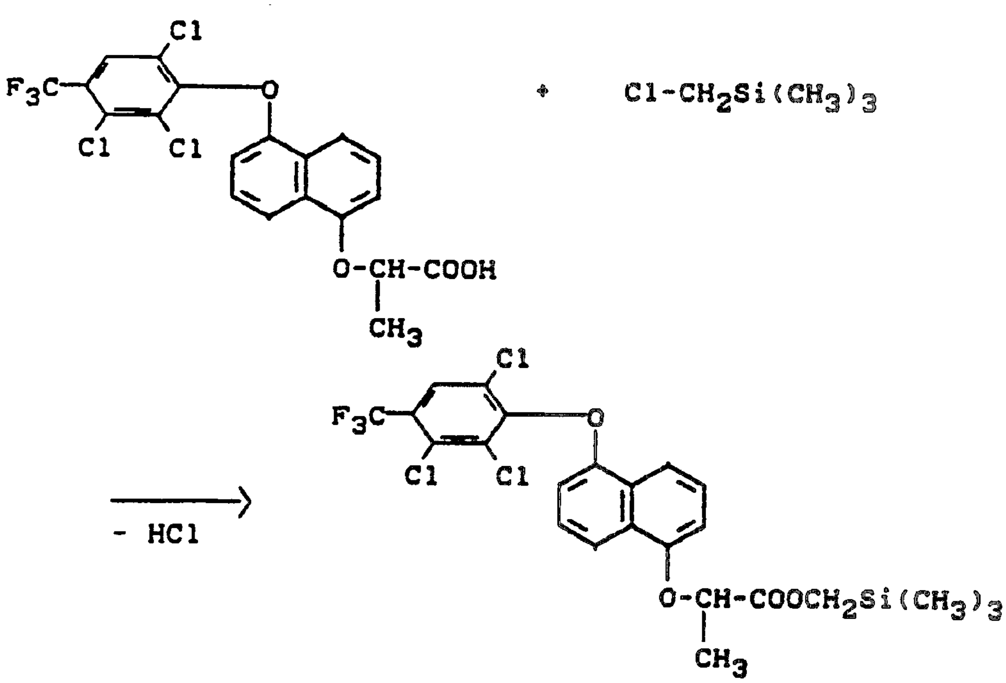


Die beim erfindungsgemäßen Verfahren (a) zur Herstellung von Verbindungen der Formel (I) als Ausgangsstoffe zu verwendenden 5-Aryloxy-1-naphthole sind durch die Formel (II) allgemein definiert.

In Formel (II) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden.

Als Beispiele für die Ausgangsstoffe der Formel (II) seien genannt :

5-(4-Trifluormethyl-phenoxy)-1-naphthol, 5-(2-Chlor-4-trifluormethyl-phenoxy)-1-naphthol, 5-(2,6-Dichlor-4-trifluormethyl-phenoxy)-1-naphthol, 5-(2-Chlor-6-fluor-4-trifluormethyl-phenoxy-1-naphthol, 5-(2,6-Dichlor-3-fluor-4-trifluormethyl-phenoxy)-1-naphthol und 5-(2,3,6-Trichlor-4-trifluormethyl-phenoxy)-1-naphthol.

Die Ausgangsstoffe der Formel (II) sind noch nicht aus der Literatur bekannt. Man erhält die Verbindungen der Formel (II), wenn man entsprechende Halogen-benzol-Derivate der allgemeinen Formel (IV)

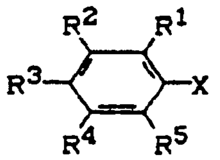

(IV)

in welcher

$R^1$, $R^2$, $R^3$, $R^4$, $R^5$ und X die oben angegebenen Bedeutungen haben,

mit 1,5-Dihydroxynaphthalin in Gegenwart eines Säureakzeptors, wie z.B. Natriumhydroxid oder Kaliumhydroxid, und in Gegenwart eines Verdünnungsmittels, wie z.B. Dimethylformamid, Dimethylacetamid, Dimethylsulfoxid, Tetramethylensulfon oder N-Methyl-pyrrolidon, bei Temperaturen zwischen 20°C und 150°C umsetzt und nach üblichen Methoden aufarbeitet.

Die Halogen-benzol-Derivate sind durch die Formel (IV) allgemein definiert. In Formel (IV) haben $R^1$, $R^2$, $R^3$, $R^4$ und $R^5$ vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden und X steht vorzugsweise für Chlor oder Fluor.

Als Beispiele für die Halogen-benzol-Derivate der Formel (IV) seien genannt:

3-Cyano-4-chlor-benzotrifluorid, 4-Chlor-benzotrifluorid, 3,4-Dichlor-benzotrifluorid, 3,4,5-Trichlor-benzotrifluorid, 3,4-Dichlor-5-fluor-benzotrifluorid, 2,3,4,5-Tetrachlor-benzotrifluorid, 3,5-Dichlor-2,4-difluor-benzotrifluorid und 3-Chlor-4,5-difluor-benzotrifluorid.

Die Verbindungen der Formel (IV) sind bekannt und/oder können nach an sich bekannten Verfahren hergestellt werden (vgl. J. Chem. Soc. 1969, 211 – 217 ; ibid. 1971, 1547 – 1549 ; EP-A 34 402 ; US-P 4 424 396; EP-A 145 314 ; FR-A 2 538 380 (Chem. Abstracts 102 (1985), 61914x)).

Die beim erfindungsgemäßen Verfahren (a) weiter als Ausgangsstoffe zu verwendenden Propionsäure-Derivate sind durch die Formel (III) allgemein definiert. In Formel (III) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde und Y steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methyl-phenylsulfonyloxy.

Als Beispiele für die Verbindungen der Formel (III) seien genannt:

α-Chlor-, α-Brom- und α-Iod-propionsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methyl-phenylsulfonyloxy)-propionsäure-methylester, -ethylester, -propylester, -butylester, -isopropylester, -isobutylester und -sec-butylester.

Unter den genannten Verbindungen der Formel (III) sind jeweils die R-Isomeren, die S-Isomeren und die racemischen Gemische dieser Isomeren zu verstehen.

Die Ausgangsstoffe der Formel (III) sind bekannt und/oder können nach an sich bekannten Verfahren hergestellt werden (vgl. DE-OS 27 58 002, DE-OS 28 54 542).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0°C und 150°C, vorzugsweise bei Temperaturen zwischen 20°C und 100°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man je Mol 5-Aryloxy-1-naphthol der Formel (II) im allgemeinen zwischen 0,5 und 1,2 Mol, vorzugsweise zwischen 0,7 und 1,0 Mol Propionsäure-Derivat der Formel (III) ein. Die Reaktionskomponenten werden im allgemeinen bei Raumtemperatur oder unter leichtem Kühlen zusammengegeben und dann – gegebenenfalls bei erhöhter Temperatur – bis zum Ende der Umsetzung gerührt.

Die Aufarbeitung kann auf übliche Weise durchgeführt werden. Beispielsweise wird das Reaktionsgemisch mit einer Säure, wie z.B. Salzsäure oder Schwefelsäure und Wasser, sowie einem mit Wasser praktisch nicht mischbaren organischen Lösungsmittel, wie z.B. Methylenchlorid oder Toluol, verrührt oder geschüttelt, die organische Phase abgetrennt, mit Wasser gewaschen, getrocknet und filtriert. Das nach Einengen des Filtrats im Rückstand verbleibende Produkt der Formel (I) kann auf übliche Weise, beispielsweise durch Säulenchromatographie, gereinigt werden.

Die beim erfindungsgemäßen Verfahren (b) als Ausgangsstoffe zu verwendenden Halogen-benzol-Derivate der Formel (IV) wurden bereits oben beschreiben.

Die beim erfindungsgemäßen Verfahren (b) weiter als Ausgangsstoffe zu verwendenden α-(5-Hydroxy-naphthalin-1-yl-oxy)-propionsäure-Derivate sind durch die Formel (V) allgemein definiert. In Formel (V) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde.

Als Beispiele für die Ausgangsstoffe der Formel (V) seien genannt :

α-(5-Hydroxy-naphthalin-1-yl-oxy)-propionsäure-, -methylester, -ethylester, -propylester, -isopropylester, -butylester, -isobutylester und -sec-butylester.

Die Ausgangsstoffe der Formel (V) sind bekannt und/oder können nach an sich bekannten Verfahren hergestellt werden (vgl. JP 79/32477, zit. in Chem. Abstracts 91 (1979), 91510j).

Verfahren (b) wird vorzugsweise unter Verwendung eines Verdünnungsmittels durchgeführt. Es kömmen vor allem diejenigen Verdünnungsmittel in Betracht, die bereits bei der Beschreibung des erfindungsgemäßen Verfahrens (a) genannt wurden. Aprotisch polare organische Lösungsmittel, wie z.B. Aceton, Acetonitril, Methylethylketon, Propionitril, Dimethylformamid, Dimethylacetamid, Dimethylsulfoxid, Sulfolan und N-Methyl-pyrrolidon werden besonders bevorzugt.

Verfahren (b) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (b) in einem größeren Bereich variiert werden. Im allgemeinen arbeitet man bei Temperaturen zwischen 0°C und 200°C, vorzugsweise bei Temperaturen zwischen 20°C und 150°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man je Mol Halogen-benzol-Derivat der Formel (IV) im allgemeinen zwischen 0,5 und 2 Mol, vorzugsweise zwischen 0,7 und 1,5 Mol, α-(5-Hydroxy-naphthalin-1-yl-oxy)-propionsäure-Derivat der Formel (V) ein.

Umsetzung und Aufarbeitung können wie oben für Verfahren (a) beschreiben durchgeführt werden.

Die beim erfindungsgemäßen Verfahren (c) als Ausgangsstoffe zu verwendenden Verbindungen sind durch die Formel (I) mit der Maßgabe, daß Z für Methoxy oder Ethoxy steht, allgemein definiert. In diesem Fall haben die Reste $R^1$, $R^2$, $R^3$, $R^4$ und $R^5$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (c) seien genannt :

α-(5-(4-Trifluormethyl-phenoxy)-, α-(5-(2-Chlor-4-trifluormethyl-phenoxy)-, α-(5-(2,6-Dichlor-4-trifluormethyl-phenoxy)-, α-(5-(2-Chlor-6-fluor-4-trifluormethylphenoxy)-, α-(5-(2,3,6-Trichlor-4-trifluormethyl-phenoxy)- und α-(5-(2,6-Dichlor-3-fluor-4-trifluormethylphenoxy)-naphthalin-1-yl-oxy)-propionsäure-methylester und -ethylester.

Die oben beschriebenen Ausgangsstoffe der Formel (I) für Verfahren (c) sind erfindungsgemäße neue Verbindungen ; sie können nach dem erfindungsgemäßen Verfahren (a) oder (b) hergestellt werden.

Verfahren (c) wird unter Verwendung von Alkalihydroxiden durchgeführt. Als Beispiel hierfür seien Lithiumhydroxid, Natriumhydroxid und Kaliumhydroxid genannt. Vorzugsweise wird Natriumhydroxid verwendet.

Verfahren (c) wird in Gegenwart von Wasser und gegebenenfalls in Gegenwart eines organischen Lösungsmittels durchgeführt. Als organische Lösungsmittel werden vorzugsweise Alkohole, wie z.B. Methanol oder Ethanol eingesetzt.

Zum Ansäuern werden bei Verfahren (c) die üblichen Mineralsäuren, wie z.B. Salzsäure oder Schwefelsäure, verwendet.

Die Reaktionstemperaturen können bei dem erfindungsgemäßen Verfahren (c) in einem größeren Bereich variiert werden. Im allgemeinen arbeitet man bei Temperaturen zwischen 10°C und 150°C, vorzugsweise bei Temperaturen zwischen 20°C und 100°C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung von Verfahren (c) werden je Mol Ausgangsverbindung der Formel (I) im allgemeinen zwischen 0,1 und 10 Mol, vorzugsweise zwischen 0,5 und 2 Mol, Alkalihydroxid eingesetzt. Die Reaktionskomponenten werden im allgemeinen bei Raumtemperatur zusammengegeben und das Reaktionsgemisch wird gegegenenfalls bei erhöhter Temperatur, bis zum Reaktionsende gerührt. Das – gegebenenfalls nach Einengen, Abkühlen und Ansäuern – kristallin anfallende Reaktionsprodukt kann durch Absaugen isoliert werden.

Die beim erfindungsgemäßen Verfahren (d) als Ausgangsstoffe zu verwendenden Verbindungen sind durch die Formel (I) mit der Maßgabe, daß Z für Hydroxy steht, allgemein definiert. In diesem Fall haben die Reste $R^1$, $R^2$, $R^2$, $R^4$ und $R^5$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (d) seien genannt :

α-(5-(4-Trifluormethyl-phenoxy)-, α-(5-(2-Chlor-4-trifluormethyl-phenoxy)-, α-(5-(2,6-Dichlor-4-trifluormethyl-phenoxy)-, α-(5-(2-Chlor-6-fluor-4-trifluormethylphenoxy)-, α-(5-(2,3,6-Trichlor-4-trifluormethyl-phenoxy)- und α-(5-(2,6-Dichlor-3-fluor-4-trifluormethylphenoxy)-naphthalin-1-yl-oxy)-propionsäure.

Die oben beschriebenen Ausgangsstoffe der Formel (I) für Verfahren (d) sind erfindungsgemäße, neue Verbindungen ; sie können nach dem erfindungsgemäßen Verfahren (c) hergestellt werden.

Verfahren (d) wird unter Verwendung eines Halogenierungsmittels durchgeführt. Es können die üblichen Mittel für die Umsetzung von Carbonsäuren zur Carbonsäurehalogeniden eingesetzt werden. Als Beispiele hierfür seien Phosgen, Thionylchlorid, Phosphorylchlorid und Benzotrichlorid genannt. Vorzugsweise wird Thionylchlorid als Halogenierungsmittel verwendet.

Verfahren (d) wird gegebenenfalls in Gegenwart eines Katalysators durchgeführt. Es können die für die Herstellung von Säurechloriden aus Säuren üblichen Katalysatoren, wie z.B. Pyridin oder Dimethylformamid verwendet werden.

Verfahren (d) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z.B. Methylenchlorid, Chloroform, Tetrachlormethan oder 1,2-Dichlorethan, in Betracht.

Die Reaktionstemperaturen können bei dem erfindungsgemäßen Verfahren (d) in einem größeren Bereich variiert werden. Im allgemeinen arbeitet man bei Temperaturen zwischen 0°C und 100°C, vorzugsweise bei Temperaturen zwischen 10°C und 90°C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (e) als Ausgangsstoffe zu verwendenden Verbindungen sind durch die Formel (I) mit der Maßgabe, daß Z für halogen steht allgemein definiert. In diesem Fall haben die Reste $R^1$, $R^2$, $R^3$, $R^4$ und $R^5$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden und Z steht vorzugsweise für Fluor, Chlor oder Brom, insbesondere für Chlor.

Als Beispiele für die Ausgangsstoffe zu Verfahren (e) seien genannt:

α-(5-(4-Trifluormethyl-phenoxy)-, α-(5-(2-Chlor-4-trifluormethyl-phenoxy)-, α-(5-(2,6-Dichlor-4-trifluormethyl-phenoxy)-, α-(5-(2-Chlor-6-fluor-4-trifluormethylphenoxy)-, α-(5-(2,3,6-Trichlor-4-trifluormethyl-phenoxy)- und α-(5-(2,6-Dichlor-3-fluor-4-trifluormethylphenoxy)-naphthalin-1-yl-oxy)-propionsäure-chlorid.

Die oben beschriebenen Ausgangsstoffe der Formel (I) für Verfahren (e) sind erfindungsgemäße, neue Verbindungen ; sie können nach dem erfindungsgemäßen Verfahren (d) hergestellt werden.

Die beim erfindungsgemäßen Verfahren (e) weiter als Ausgangsstoffe einzusetzenden Verbindungen sind durch die Formel (VI) allgemein definiert. In Formel (VI) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde.

Als Beispiele für die Ausgangsstoffe der Formel (VI) seien genannt:

Methylamin, Ethylamin, Propylamin, Isopropylamin, Anilin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxy-ethanol, 3-Benzyloxy-propanol, 2-Benzylthio-ethanol, Hydroxymethanphosphonsäure-diethylester und -dimethylester, 1-Hydroxy-ethan-phosphonsäure-dimethylester und -diethylester, 1-Hydroxy-1-phenyl-methanphosphonsäure-dimethylester und -diethylester, Acetonoxim, 3-Hydroxyfuran, Furfurylalkohol, Perhydrofurfurylalkohol, Milchsäure-methylester und - ethylester und Glycolsäure-methylester und -ethylester.

Diese Verbindungen sind bekannte Synthesechemikalien.

Verfahren (e) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden.

Verfahren (e) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (e) in einem größeren Bereich variiert werden. Im allgemeinen arbeitet man bei Temperaturen zwischen −20°C und +100°C, vorzugsweise bei Temperaturen zwischen 0°C und 50°C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (e) jeweils nach üblichen Methoden. Beispielsweise wird das Reaktionsgemisch – gegebenenfalls nach Einengen - mit Wasser verdünnt und das gewünschte Reaktionsprodukt wird mit einem mit Wasser praktisch nicht mischbaren organischen Lösungsmittel, z.B. Methylenchlorid, Chloroform, Diethylether, Toluol oder Xylol, extrahiert. Die organische Extraktionslösung wird mit Wasser gewaschen, mit einem üblichen Trockenmittel, wie z.B. Natriumsulfat, getrocknet und filtriert. Nach dem Einengen des Filtrats erhält man die Verbindungen der Formel (I) als Rohprodukte, welche auf übliche Weise, z.B. chromatographisch und/oder durch Umkristallisation gereinigt werden können.

Die beim erfindungsgemäßen Verfahren (f) als Ausgangsstoffe zu verwendenden Verbindungen sind durch die Formel (I) mit der Maßgabe, daß Z für Hydroxy steht, allgemein definiert. In diesem Fall haben die Reste $R^1$, $R^2$, $R^3$, $R^4$ und $R^5$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe der Formel (I) zu Verfahren (f) seien genannt:

α-(5-(4-Trifluormethyl-phenoxy)-, α-(5-(2-Chlor-4-trifluormethyl-phenoxy)-, α-(5-(2,6-Dichlor-4-trifluormethyl-phenoxy)-, α-(5-(2-Chlor-6-fluor-4-trifluormethyl-phenoxy)-, α-(5-(2,3,6-Trichlor-4-trifluormethyl-phenoxy)- und α-(5-(2,6-Dichlor-3-fluor-4-trifluormethyl-phenoxy)-naphthalin-1-yl-oxy)-propionsäure.

Die oben beschriebenen Ausgangsstoffe der Formel (I) für Verfahren (f) sind erfindungsgemäße, neue Verbindungen ; sie können nach dem erfindungsgemäßen Verfahren (c) hergestellt werden.

Die beim erfindungsgemäßen Verfahren (f) weiter als Ausgangsstoffe einzusetzenden Verbindungen sind durch die Formel (VII) allgemein definiert. In Formel (VII) stehen vorzugsweise $R^6$ für $C_1$-$C_8$-Alkyl, $C_3$-$C_4$-Alke-

nyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl und $X^1$ für Chlor, Brom oder lod.

Insbesondere stehen in Formel (VII) $R^6$ für Trimethylsilylmethyl und $X^1$ für Chlor.

Trimethylsilylmethylchlorid wird als Ausgangsverbindung der Formel (VII) für Verfahren (f) besonders bevorzugt.

Die Ausgangstoffe der Formel (VII) sind bekannte Synthesechemikalien.

Verfahren (f) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden. Aceton, Acetonitril und Dimethylformamid werden besonders bevorzugt.

Verfahren (f) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden. 1,8-Diazabicyclo-[5.4.0]-undec-7-en (DBU) wird besonders bevorzugt.

Die Reaktionstemperaturen können bei dem erfindungsgemäßen Verfahren (f) in einem größeren Bereich variiert werden. Im allgemeinen arbeitet man bei Temperaturen zwischen 0°C und 150°C, vorzugsweise bei Temperaturen zwischen 20°C und 100°C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung von Verfahren (f) setzt man je Mol Ausgangsverbindung der Formel (I) im allgemeinen zwischen 1 und 3 Mol, vorzugsweise zwischen 1,1 und 2,5 Mol Ausgangsverbindung der Formel (VII) ein.

Umsetzung und Aufarbeitung können wie oben für Verfahren (a) beschrieben durchgeführt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden :

Dikotyle Unkräuter der Gattungen :

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen :

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen :

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen :

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wege und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung monokotyler Unkräuter in monokotylen und in dikotylen Kulturen, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffe-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage :

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln ; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage : z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide ; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage, ferner auch 2,4-Dichlorphenoxyessigsäure (2,4-D) ; 4-(2,4-Dichlorphenoxy)buttersäure (2,4-DB) ; 2,4-Dichlorphenoxypropionsäure (2,4-DP) ; 5-(2-Chlor-4-trifluormethyl-phenoxy)-2-nitrobenzoesäure (ACIFLUORFEN) ; Methyl-5-(2,4-dichlorphenoxy)-2-nitrobenzoat (BIFENOX) ; 3,5-Dibrom-4-hydroxy-benzonitril (BROMOXYNIL) ; Ethyl-2-{[(4-chlor-6-methoxy-2-pyrimidinyl)-aminocarbonyl]-aminosulfonyl}-benzoat (CHLORIMURON) ; 2-Chlor-N-{[(4-methoxy-6-methyl-1,3,5-triazin-2-yl)-amino]-carbonyl}-benzolsulfonamid (CHLORSULFURON) ; N,N-Dimethyl-N'-(3-chlor-4-methylphenyl)harnstoff (CHLORTOLURON) ; N,N-Dimethyl-N'-(3-trifluormethylphenyl)-harnstoff (FLUOMETURON) ; 5-(2-Chlor-4-trifluormethyl-phenoxy)-N-methylsulfonyl-2-nitrobenzamid (FOMESAFEN) ; Methyl-2-[4,5-dihydro-4-methyl-4-(1-methyl-ethyl)-5-oxo-1H-imidazol-2-yl]-4(5)-methylbenzoat (IMAZAMETHABENZ) ; 2-[5-Methyl-5-(1-methylethyl)-4-oxo-2-imidazolin-2-yl]-3-chinolincarbonsäure (IMAZAQUIN) ; 2-[4,5-Dihydro-4-methyl-4-isopropyl-5-oxo-(1H)-imidazol-2-yl]-5-ethyl-pyridin-3- carbonsäure (IMAZETHAPYR); 3,5-Di-iod-4-hydroxybenzonitril (IOXYNIL) ; N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN) ; (2-Methyl-4-chlorphenoxy)-essigsäure (MCPA) ; (4-Chlor-2-methylphenoxy)-propionsäure (MCPP) ; N-Methyl-2-(1,3-benzthiazol-2-yloxy)-acetanilid (MEFENACET) ; 2-{[[((4-Methoxy-6-methyl-1,3,5-triazin-2-yl)-amino)-carbonyl]-amino]-sulfonyl}-benzoesäure oder deren

Methylester (METSULFURON) ; (2-Chlor-4-trifluormethylphenyl)-(3-ethoxy-4-nitro-phenyl)-ether (OXYFLUORFEN) ; N-(1-Ethylpropyl)-3,4-dimethyl-2,6-dinitroanilin (PENDIMETHALIN) ; 3-[[[(4-Methoxy-6-methyl-1,3,5-triazin-2-yl)-amino]-carbonyl]-amino]-sulfonyl]-thiophen-2- carbonsäure-methylester (THIAMETURON) ; 3,5,6-Trichlor-2-pyridyloxyessigsäure (TRICLOPYR) und 2,6-Dinitro-4-trifluormethyl-N,N-dipropylanilin (TRIFLURALIN), sowie 4-Amino-6-tert.-butyl-3-ethylthio-1,2,4-triazin-5-(4H)-on (ETHIOZIN), 3-Isopropyl-2,1,3-benzothiadiazin-4-on-2,2-dioxid (BENTAZON), N-Phosphonomethylglycin (GLYPHOSPHATE) and O-(6-Chlor-3-phenylpyridazin-4-yl)-S-octyl-thiocarbonat (PYRIDATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

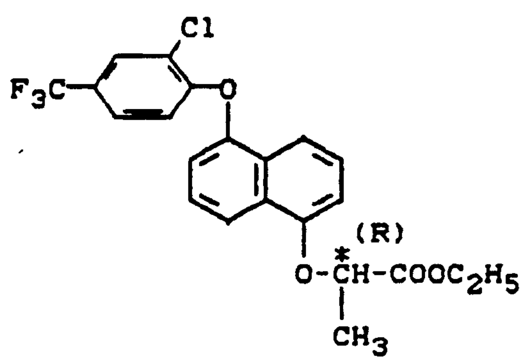

Eine Mischung aus 11,6 g (0,03 Mol) 5-(2-Chlor-4-tri-fluormethyl-phenoxy)-1-naphthol, 5,4 g (0,02 Mol) (S)-α-(4-Methyl-phenylsulfonyloxy)-propionsäure-ethylester, 4,2 g Kaliumcarbonat und 100 ml Acetonitril wird 15 Stunden unter Rückfluß zum Sieden erhitzt. Nach Abkühlen auf 20°C wird das Reaktionsgemisch mit 2N-Salzsäure angesäuert und mit Toluol geschüttelt. Die organische Phase wird abgetrennt, mit Wasser gewaschen, mit Natriumsulfat getrocknet und filtriert. Vom Filtrat wird das Lösungsmittel im Wasserstrahlvakuum sorgfältig abdestilliert. Der Rückstand wird durch Säulenchromatographie (Toluol/Hexan an Kieselgel) gereinigt.

Man erhält 1,6 g (19% der Theorie) (R)-α-(5-(2-Chlor-4-trifluormethyl-phenoxy)-naphthalin-1-yl-oxy)-propionsäure-ethylester als öligen Rückstand vom Brechungsindex $n_D^{20}$ = 1,5611.

Analog Beispiel 1 und entsprechend der allgemeinen Beschreibung der erfindungsgemäßen Herstellungsverfahren können die in der nachstehenden Tabelle 1 aufgeführten Verbindungen der Formel (I) erhalten werden.

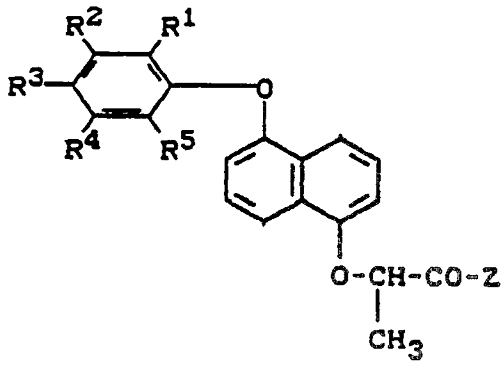

(I)

Tabelle 1: Beispiele für die Verbindungen der Formel (I)

| Bsp.-Nr. | $R^1$ | $R^2$ | $R^3$ | $R^4$ | $R^5$ | Z | Physikal. Daten |
|---|---|---|---|---|---|---|---|
| 2 | H | H | $CF_3$ | H | H | $OC_2H_5$ | $n_D^{20}=1,5728$ |
| 3 | Cl | H | $CF_3$ | H | Cl | $OC_2H_5$ | (amorph) |
| 4 | Cl | H | $CF_3$ | H | F | $OCH_3$ | |
| 5 | Cl | Cl | $CF_3$ | H | Cl | $OC_3H_7$ | |
| 6 | Cl | F | $CF_3$ | H | Cl | $OC_4H_9$ | |
| 7 | H | H | $CF_3$ | H | H | OH | |
| 8 | Cl | H | $CF_3$ | H | H | OH | |
| 9 | Cl | H | $CF_3$ | H | F | OH | |
| 10 | Cl | Cl | $CF_3$ | H | Cl | OH | |
| 11 | Cl | F | $CF_3$ | H | Cl | OH | |

Tabelle 1 - Fortsetzung

| Bsp.-Nr. | $R^1$ | $R^2$ | $R^3$ | $R^4$ | $R^5$ | Z | Physikal. Daten |
|---|---|---|---|---|---|---|---|
| 12 | Cl | H | $CF_3$ | H | H | Cl | |
| 13 | Cl | H | $CF_3$ | H | Cl | OH | |
| 14 | Cl | H | $CF_3$ | H | Cl | Cl | |
| 15 | Cl | Cl | $CF_3$ | H | Cl | Cl | |
| 16 | Cl | H | $CF_3$ | H | Cl | $OCH(CH_3)_2$ | |
| 17 | Cl | H | $CF_3$ | H | H | $OC_4H_9$ | |
| 18 | Cl | H | $CF_3$ | H | Cl | $OCH_2COOCH_3$ | |
| 19 | Cl | H | $CF_3$ | H | H | $OCH_2COOC_4H_9$ | |
| 20 | Cl | H | $CF_3$ | H | F | $OCHCOOC_2H_5$<br>\|<br>$CH_3$ | |
| 21 | Cl | H | $CF_3$ | H | Cl | $SCH_2COOCH_3$ | |
| 22 | Cl | Cl | $CF_3$ | H | Cl | $OCH_2CH_2OCH_3$ | |
| 23 | Cl | H | $CF_3$ | H | Cl | $OCH_2CH_2SC_2H_5$ | |
| 24 | Cl | H | $CF_3$ | H | H | O<br>‖<br>$OCH_2P(OC_2H_5)_2$ | |
| 25 | Cl | H | $CF_3$ | H | H | O<br>‖<br>$OCH-P(OCH_3)_2$<br>\|<br>$C_6H_5$ | |
| 26 | Cl | Cl | $CF_3$ | H | Cl | $SC_2H_5$ | |
| 27 | Cl | H | $CF_3$ | H | Cl | $NHCH(CH_3)_2$ | |
| 28 | Cl | H | $CF_3$ | H | H | $N(C_2H_5)_2$ | |

Tabelle 1 - Fortsetzung

| Bsp.-Nr. | $R^1$ | $R^2$ | $R^3$ | $R^4$ | $R^5$ | Z | Physikal. Daten |
|---|---|---|---|---|---|---|---|
| 29 | Cl | H | $CF_3$ | H | Cl | $OCH_2CH_2OCH_2-$ 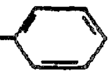 | |
| 30 | Cl | H | $CF_3$ | H | H | $OCH_2$ 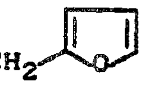 | |
| 31 | Cl | H | $CF_3$ | H | H | $NHOCH_3$ | |
| 32 | Cl | H | $CF_3$ | H | H | $NHOC_2H_5$ | |
| 33 | Cl | H | $CF_3$ | H | Cl | $NHCH_2COOC_2H_5$ | |
| 34 | Cl | H | $CF_3$ | H | H | $NHNHSO_2CH_3$ | |
| 35 | Cl | H | $CF_3$ | H | Cl | $OCH_2Si(CH_3)_3$ | |
| 36 | CN | H | $CF_3$ | H | H | $OC_2H_5$ | (amorph) |
| 37 | Cl | H | $CF_3$ | H | Cl | $O(CH_2)_3OCH_2-$ 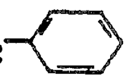 | |

Ausgangsstoffe der Formel (II)

Beispiel (II-1)

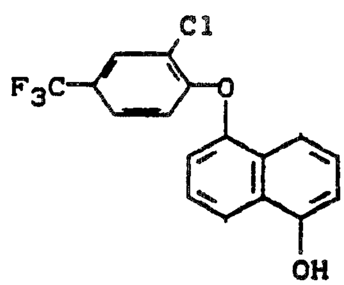

20,4 g (0,095 Mol) 3,4-Dichlor-benzotrifluorid werden zu einer auf 120°C erhitzten Mischung aus 28,4 g (0,18 Mol) 1,5-Dihydroxy-naphthalin, 10,1 g (0,18 Mol) Kaliumhydroxid-Pulver und 100 ml Dimethylsulfoxid langsam unter Rühren gegeben und das Reaktionsgemisch wird 10 Stunden bei 120°C gerührt. Nach Abkühlen auf 20°C wird mit 2N-Salzsäure angesäuert und mit Toluol verrührt. Das kristallin angefallene Produkt wird durch Absaugen isoliert und verworfen (1,5-Dihydroxynaphthalin). Die Toluolphase wird abgetrennt und mit Natriumsulfat getrocknet. Nach Filtration wird das Lösungsmittel im Vakuum sorgfältig abdestilliert.

Man erhält 11,6 g (36% der Theorie) 5-(2-Chlor-4-trifluormethyl-phenoxy)-1-naphthol als amorphen Rückstand ($^1$H-NMR ($CDCl_3$), δ ($R^2$) : 7,3 ppm, wenn $R^1$ = Cl, $R^3$ = $CF_3$).

Analog Beispiel (II-1) können die in der nachstehenden Tabelle 2 aufgeführten Ausgangsstoffe der Formel (II) hergestellt werden.

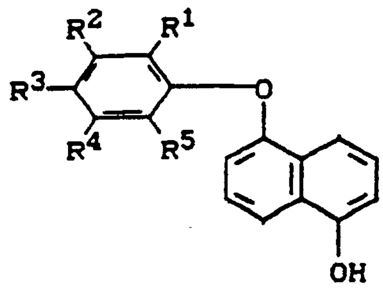

(II)

Tabelle 2: Beispiele für die Ausgangstoffe der Formel (II)

| Beisp.-Nr. | $R^1$ | $R^2$ | $R^3$ | $R^4$ | $R^5$ | Physikalische Daten |
|---|---|---|---|---|---|---|
| II-2 | H | H | $CF_3$ | H | H | |
| II-3 | Cl | H | $CF_3$ | H | Cl | |
| II-4 | Cl | Cl | $CF_3$ | H | Cl | |
| II-5 | Cl | F | $CF_3$ | H | Cl | |
| II-6 | Cl | H | $CF_3$ | H | F | |
| II-7 | CN | H | $CF_3$ | H | H | |

Anwendungsbeispiele

In den folgenden Anwendungsbeispielen wird die nachstehend aufgeführte Verbindung als Vergleichssubstanz herangezogen :

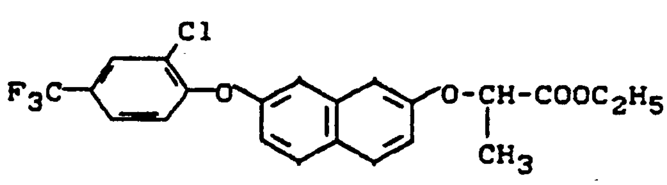

(A)

α-(7-(2-Chlor-4-trifluormethyl-phenoxy)-naphthalin-2-yl-oxy)-propionsäure-ethylester
(bekannt aus EP-A 179 015/Beispiel 1).

Beispiel A

Post-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten :
0% = keine Wirkung (wie unbehandelte Kontrolle)
100% = totale Vernichtung

In diesem Test zeigt beispielsweise die Verbindung gemäß Herstellungsbeispiel (1) bei guter Selektivität in Soja, Baumwolle und Weizen erheblich stärkere Wirkung gegen Problemunkräuter, wie z.B. Digitaria, Panicum und Setaria, als die Vergleichssubstanz (A).

**Ansprüche**

1. α-(5-Aryloxy-naphtalin-1-yl-oxy)-propionsäure-Derivate der Formel (I),

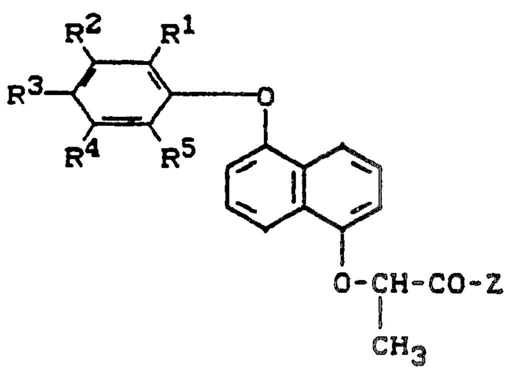

(I)

in welcher

$R^1$ für Wasserstoff, Halogen, Cyano oder Trifluormethyl steht,
$R^2$ für Wasserstoff oder Halogen steht,
$R^3$ für Halogen, Cyano, Trifluormethyl oder Trifluormethoxy steht,
$R^4$ für Wasserstoff oder Halogen steht,
$R^5$ für Wasserstoff oder Halogen steht und
Z für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung –O-$R^6$ steht, worin

$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl, Alkylidenamino oder für ein Ammonium-, Alkylammonium-, Alkalimetall- oder Erdalkalimetall-äquivalent steht oder für die Gruppierung

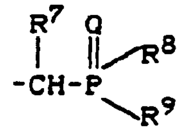

steht, worin

$R^7$ für Wasserstoff, Alkyl, Aryl, Furyl, Thienyl oder Pyridyl steht,

$R^8$ für Alkyl oder Alkoxy steht,

$R^9$ für Alkoxy steht und

Q für Sauerstoff oder Schwefel steht, oder

$R^6$ für die Gruppierung $-(CH_2)_n-R^{10}$ steht, worin

$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht un d

n für die Zahlen 0, 1 oder 2 steht.

2. α-5-(Aryloxy-naphthalin-1-yl-oxy)-propionsäure-Derivate der Formel (I) gemäß Anspruch 1, in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Cyano, Trifluormethyl oder Trifluormethoxy steht,

$R^4$ für Wasserstoff, Fluor oder Chlor steht,

$R^5$ für Wasserstoff, Fluor, Chlor oder Brom steht und

Z für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung $-O-R^6$ steht, worin

$R^6$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium- oder Calciumäquivalent steht, oder für die Gruppierung

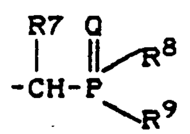

steht, worin

$R^7$ für Wasserstoff, $C_1$-$C_4$-Alkyl, Phenyl, Furyl, Thienyl oder Pyridyl steht,

$R^8$ für $C_1$-$C_4$-Alkyl oder $C_1$-$C_4$-Alkoxy steht,

$R^9$ für $C_1$-$C_4$-Alkoxy steht und

Q für Sauerstoff oder Schwefel steht, oder

$R^6$ für die Gruppierung $-(CH_2)_n-R^{10}$ steht, worin

n für die Zahlen 0, 1 oder 2 steht und

$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

3. α-(5-Aryloxy-naphthalin-1-yl-oxy)-propionsäure-Derivate der Formel (I) gemäß Anspruch 1, in welcher

$R^1$ für Wasserstoff, Cyano, Fluor oder Chlor steht,

$R^2$ für Wasserstoff, Fluor oder Chlor steht,

$R^3$ für Trifluormethyl steht,

$R^4$ für Wasserstoff, Fluor oder Chlor steht,

$R^5$ für Wasserstoff, Fluor oder Chlor steht und

Z für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilymethyl oder für ein Ammonium-, $C_1$-$C_3$-Alkylammonium-, Natrium-, oder Kaliumäquivalent steht, oder für die Gruppierung

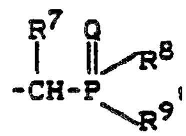

steht, worin

$R^7$ für Wasserstoff, Methyl, Phenyl, Furyl, Thienyl oder Pyridyl steht,

$R^8$ für Methoxy oder Ethoxy steht,

$R^9$ für Methoxy oder Ethoxy steht und

Q für Sauerstoff oder Schwefel steht

oder

$R^6$ für die Gruppierung $(-CH_2-)_n-R^{10}$ steht, worin

n für die Zahlen 0, 1 oder 2 steht und

$R^{10}$ für einen gegebenenfalls durch Chlor und/oder Methyl subtituierten heterocyclischen Rest aus der Reihe Furyl, Tetrahydrofuryl, Thienyl, Perhydropyranyl, Oxazolyl, Thiazolyl und Dioxolanyl steht.

4. R-Enantiomere der Formel (I) gemäß Anspruch 3.

5. Verfahren zur Herstellung der α-(5-Aryloxynaphthalin-1-yl-oxy)-propionsäure-Derivate der Formel (I),

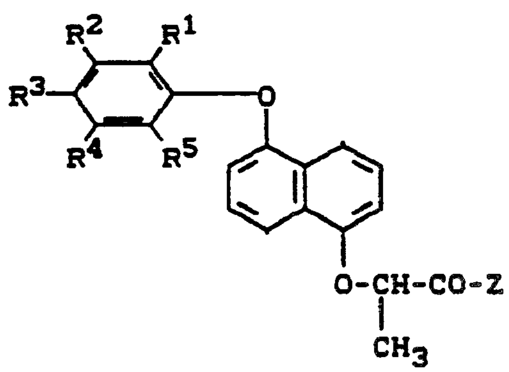

(I)

in welcher

$R^1$ für Wasserstoff, Halogen, Cyano oder Trifluormethyl steht,

$R^2$ für Wasserstoff oder Halogen steht,

$R^3$ für Halogen, Cyano, Trifluormethyl oder Trifluormethoxy steht,

$R^4$ für Wasserstoff oder Halogen steht,

$R^5$ für Wasserstoff oder Halogen steht und

Z für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung $-O-R^6$ steht, worin

$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl, Alkylidenamino oder für ein Ammonium-, Alkylammonium-, Alkalimetall- oder Erdalkalimetall-äquivalent steht oder für die Gruppierung

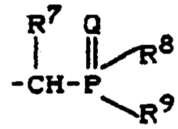

steht, worin

$R^7$ für Wasserstoff, Alkyl, Aryl, Furyl, Thienyl oder Pyridyl steht,

$R^8$ für Alkyl oder Alkoxy steht,

$R^9$ für Alkoxy steht und

Q für Sauerstoff oder Schwefel steht,

oder

$R^6$ für die Gruppierung $-(CH_2)_n-R^{10}$ steht, worin

$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und

n für die Zahlen 0, 1 oder 2 steht,

dadurch gekennzeichnet, daß man

(a) 5-Aryloxy-1-naphthole der allgemeinen Formel (II)

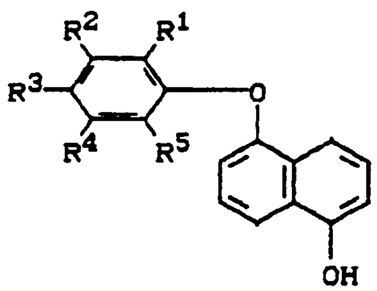

(II)

in welcher

$R^1$, $R^2$, $R^3$, $R^4$ und $R^5$ die oben angegebenen Bedeutungen haben,

mit Propionsäure-Derivaten der allgemeinen Formel (III)

$$Y - \underset{\displaystyle CH_3}{\underset{|}{CH}} - CO - Z \quad (III)$$

in welcher

Z die oben angegebene Bedeutung hat und

Y für eine nucleophile Abgangsgruppe steht, gegebenenfalls in Gegenwart eines Säureakzeptors und gegebenenfalls in Gegenwart eines Verdünnungsmittels umsetzt, oder daß man

(b) Halogen-benzol-Derivate der allgemeinen Formel (IV)

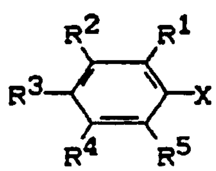

(IV)

in welcher

$R^1$, $R^2$, $R^3$, $R^4$ und $R^5$ die oben angegebenen Bedeutungen haben und

X für Halogen steht,

mit α-(5-Hydroxy-naphthalin-1-yl-oxy)-propionsäure-Derivaten der allgemeinen Formel (V)

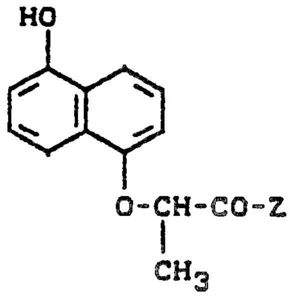

(V)

in welcher
Z die oben angegebene Bedeutung hat,
gegebenenfalls in Gegenwart eines Säureakzeptors und gegebenenfalls in Gegenwart eines Verdünnungsmittels umsetzt, oder daß man
(c) für den Fall, daß Z für Hydroxy steht und die Reste $R^1$ bis $R^5$ die oben angegebenen Bedeutungen haben, Verbindungen der allgemeinen Formel (I), in welcher Z für Methoxy oder Ethoxy steht und die Reste $R^1$ bis $R^5$ die oben angegebenen Bedeutungen haben,
mit einem Alkalihydroxid in Gegenwart von Wasser und gegebenenfalls in Gegenwart eines organischen Lösungsmittels umsetzt und dann – gegebenenfalls nach Einengen – mit einer Mineralsäure ansäuert, oder
(d) für den Fall, daß Z für Halogen steht und die Reste $R^1$ bis $R^5$ die oben angegebenen Bedeutungen haben, Verbindungen der allgemeinen Formel (I), in welcher Z für Hydroxy steht und die Reste $R^1$ bis $R^5$ die oben angegebenen Bedeutungen haben,
mit einem Halogenierungsmittel gegebenenfalls in Gegenwart eines Katalysators und gegebenenfalls in Gegenwart eines Verdünnungsmittels umsetzt, oder
(e) für den Fall, daß Z mit Ausnahme von Halogen die oben angegebene Bedeutung hat und Reste $R^1$ bis $R^5$ die oben angegebenen Bedeutun haben, Verbindungen der allgemeinen Formel (I), in welcher Z für Halogen steht und die Reste $R^1$ bis $R^5$ die oben angegebenen Bedeutungen haben,
mit Verbindungen der allgemeinen Formel (VI)

$$H-Z \quad (VI)$$

in welcher
Z mit Ausnahme von Halogen die oben angegebene Bedeutung hat,
gegebenenfalls in Gegenwart eines Säureakzeptors und gegebenenfalls in Gegenwart eines Verdünnungsmittels umsetzt, oder
(f) für den Fall, daß Z für die Gruppierung $-O-R^6$ steht, worin $R^6$ mit Ausnahme von Ammonium, Alkylammonium, Alkalimetall und Erdalkalimetall die oben angegebene Bedeutung hat und die Reste $R^1$ bis $R^5$ die oben angegebenen Bedeutungen haben, Verbindungen der allgemeinen Formel (I), in wel Z für Hydroxy steht und die Reste $R^1$ bis $R^5$ die oben angegebenen Bedeutungen haben,
mit Verbindungen der allgemeinen Formel (VII)

$$X^1-R^6 \quad (VII)$$

in welcher
$R^6$ mit Ausnahme von Ammonium, Alkylammonium, Alkalimetall und Erdalkalimetall die oben angegebenen Bedeutungen hat und
$X^1$ für Halogen steht,
gegebenenfalls in Gegenwart eines Säureakzeptors und gegebenenfalls in Gegenwart eines Verdünnungsmittels umsetzt.

6. Herbizide Mittel, gekennzeichnet durch einen Gehalt an mindestens einem α-(5-Aryloxy-naphthalin-1-yl-oxy)-propionsäure-Derivat der Formel (I) gemäß den Ansprüchen 1 bis 5.

7. Verfahren zur Bekämpfung von Unkräutern, dadurch gekennzeichnet, daß man α-(5-Aryloxy-naphthalin-1-yl-oxy)-propionsäure-Derivate der Formel (I) gemäß den Ansprüchen 1 bis 5 auf die Unkräuter und/oder ihren Lebensraum einwirken läßt.

8. Verwendung von α-(5-Aryloxy-naphthalin-1-yl-oxy)-propionsäure-Derivaten der Formel (I) gemäß den Ansprüchen 1 bis 5 zur Bekämpfung von Unkräutern.

9. Verfahren zur Herstellung von herbiziden Mitteln, dadurch gekennzeichnet, daß man α-(5-Aryloxy naphthalin-1-yl-oxy)-propionsäure-Derivate der Formel (I) gemäß den Ansprüchen 1 bis 5 mit Streckmitteln und/oder oberflächenaktiven Substanzen vermischt.

10. 5-Aryloxy-1-naphthole der Formel (II)

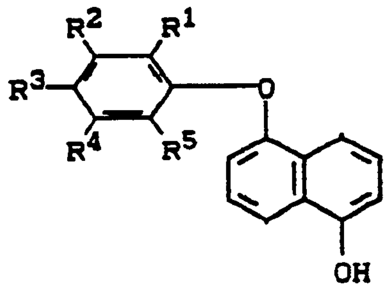

(II)

in welcher

$R^1$ für Wasserstoff, Halogen, Cyano oder Trifluormethyl steht,

$R^2$ für Wasserstoff oder Halogen steht,

$R^3$ für Halogen, Cyano, Trifluormethyl oder Trifluormethoxy steht,

$R^4$ für Wasserstoff oder Halogen steht,

$R^5$ für Wasserstoff oder Halogen steht.

## Claims

1. α-(5-Aryloxy-naphtalen-l-yl-oxy)-propionic acid derivatives of the formula (I)

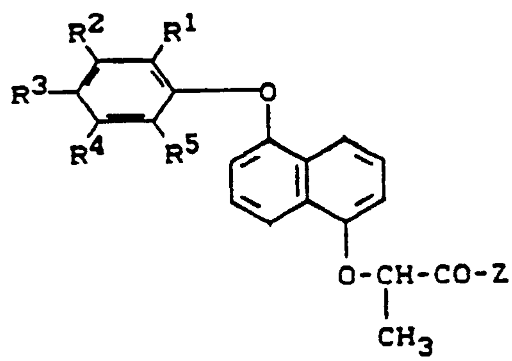

(I)

in which

$R^1$ represents hydrogen, halogen, cyano or trifluoromethyl,

$R^2$ represents hydrogen or halogen,

$R^3$ represents halogen, cyano, trifluoromethyl or trifluoromethoxy,

$R^4$ represents hydrogen or halogen,

$R^5$ represents hydrogen or halogen and Z represents halogen, hydroxyl, amino, alkylamino, alkenylamino, alkinylamino, arylamino, aralkylamino, alkoxycarbonylalkylamino, cyanamido, dialkylamino, dialkenylamino, alkylsulphonylamino, arylsulphonylamino, hydroxylamino, alkoxyamino, hydrazino, alkylsulphonylhydrazino, arylsulphonylhydrazino, alkylthio, arylthio, aralkylthio, alkoxycarbonylalkylthio or the –O-$R^6$ group,

wherein

$R^6$ represents an optionally halogen-substituted radical from the series comprising alkyl, alkenyl, alkinyl, alkoxyalkyl, alkylthioalkyl, alkylsulphinylalkyl, alkylsulphonylalkyl, aryloxyalkyl, trialkylsilylalkyl, arylthioalkyl,

aralkoxyalkyl, aralkylthioalkyl, alkoxycarbonylalkyl, alkylaminocarbonylalkyl, aralkyl, azolylalkyl, alkylideneamino or an ammonium equivalent, an alkylammonium equivalent, an alkali metal equivalent or an alkaline earth metal equivalent or the group

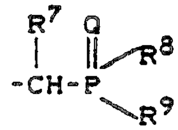

wherein

$R^7$ represents hydrogen, alkyl, aryl, furyl, thienyl or pyridyl,

$R^8$ represents alkyl or alkoxy,

$R^9$ represents alkoxy and

Q represents oxygen or sulphur,

or

$R^6$ represents the $-(CH_2)_n-R^{10}$ group,

wherein

$R^{10}$ represents a heterocyclic radical from the series comprising furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl, each of which is optionally substituted by halogen and/or alkyl, and

n represents the numbers 0, 1 or 2.

2. α-5-(aryloxy-naphthalen-1-yl-oxy)-propionic acid derivatives of the formula (I) according to Claim 1, in which

$R^1$ represents hydrogen, fluorine, chlorine, bromine, cyano or trifluoromethyl,

$R^2$ represents hydrogen, fluorine or chlorine,

$R^3$ represents fluorine, chlorine, bromine, cyano, trifluoromethyl or trifluoromethoxy,

$R^4$ represents hydrogen, fluorine or chlorine,

$R^5$ represents hydrogen, fluorine, chlorine or bromine and

Z represents chlorine, hydroxyl, amino, $C_1$-$C_6$-alkylamino, $C_3$-$C_4$-alkenylamino, $C_3$-$C_4$-alkinylamino, phenylamino, benzylamino, $C_1$-$C_4$-alkoxycarbonyl-$C_1$-$C_2$-alkylamino, cyanamino, di-($C_1$-$C_4$-alkyl)-amino, di-($C_3$-$C_4$-alkenyl)-amino, $C_1$-$C_4$-alkylsulphonylamino, phenylsulphonylamino, tolylsulphonylamino, hydroxylamino, $C_1$-$C_6$-alkoxyamino, hydrazino, $C_1$-$C_4$-alkylsulphonylhydrazino, phenylsulphonylhydrazino, tolylsulphonylhydrazino, $C_1$-$C_4$-alkylthio, phenylthio, benzylthio, $C_1$-$C_4$-alkoxy-carbonyl-$C_1$-$C_2$-alkylthio or the –O-$R^6$ group,

wherein

$R^6$ represents a radical from the series comprising $C_1$-$C_6$-alkyl, $C_3$-$C_4$-alkenyl, $C_3$-$C_4$-alkinyl, $C_1$-$C_4$-alkoxy-$C_1$-$C_4$-alkyl, $C_1$-$C_4$-alkylthio-$C_1$-$C_4$-alkyl, $C_1$-$C_4$-alkylsulphinyl-$C_1$-$C_4$ alkyl, $C_1$-$C_4$-alkylsulphonyl-$C_1$-$C_4$-alkyl, phenoxy-$C_1$-$C_3$-alkyl, trimethylsilylmethyl, phenylthio-$C_1$-$C_3$-alkyl, benzyl-oxy-$C_1$-$C_3$-alkyl, benzylthio-$C_1$-$C_3$-alkyl, $C_1$-$C_4$-alkoxycarbonyl-$C_1$-$C_2$-alkyl, $C_1$-$C_4$-alkylaminocarbonyl-$C_1$-$C_2$-alkyl, benzyl, pyrazolyl-$C_1$-$C_4$-alkyl or $C_2$-$C_4$-alkylideneamino, each of which is optionally substituted by fluorine and/or chlorine, or represents an ammonium equivalent, a $C_1$-$C_4$-alkylammonium equivalent, a sodium equivalent, potassium equivalent or calcium equivalent, or the group

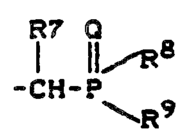

wherein

$R^7$ represents hydrogen, $C_1$-$C_4$-alkyl, phenyl, furyl, thienyl or pyridyl,

$R^8$ represents $C_1$-$C_4$-alkyl or $C_1$-$C_4$-alkoxy,

$R^9$ represents $C_1$-$C_4$-alkoxy and

Q represents oxygen or sulphur,

or

$R^6$ represents the $-(CH_2)_n-R^{10}$ group,

wherein

n represents the numbers 0, 1 or 2 and $R^{10}$ represents a heterocyclic radical from the series comprising furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl, each of which is optionally substituted by fluorine, chlorine, bromine and/or $C_1$-$C_4$-alkyl.

3. α-(5-Aryloxy-naphthalen-1-yl-oxy)-propionic acid derivatives of the formula (I) according to Claim 1, in which

$R^1$ represents hydrogen, cyano, fluorine or chlorine,

$R^2$ represents hydrogen, fluorine or chlorine,

$R^3$ represents trifluoromethyl,

$R^4$ represents hydrogen, fluorine or chlorine,

$R^5$ represents hydrogen, fluorine or chlorine and

Z represents chlorine, hydroxyl, amino, $C_1$-$C_4$-alkylamino, phenylamino, $C_1$-$C_4$-alkoxy-carbonyl-$C_1$-$C_2$-alkylamino, di-($C_1$-$C_3$-alkyl)-amino, $C_1$-$C_4$-alkylsulphonylamino, phenylsulphonylamino, hydroxylamino, cyanamido, $C_1$-$C_4$-alkoxyamino, hydrazino, $C_1$-$C_4$-alkylsulphonylhydrazino, phenylsulphonylhydrazino, $C_1$-$C_4$-alkylthio or $C_1$-$C_4$-alkoxy-carbonyl-$C_1$-$C_2$-alkylthio or the –O-$R^6$ group,

wherein

$R^6$ represents $C_1$-$C_4$-alkyl, $C_1$-$C_2$-alkoxy-$C_1$-$C_2$-alkyl,$C_1$-$C_2$-alkylthio-$C_1$-$C_2$-alkyl, $C_1$-$C_2$-alkylsulphinyl-$C_1$-$C_2$-alkyl, $C_1$-$C_2$-alkylsulphonyl-$C_1$-$C_2$-alkyl, benzyloxy-$C_1$-$C_3$-alkyl, benzylthio-$C_1$-$C_3$-alkyl, $C_1$-$C_4$-alkoxycarbonyl-$C_1$-$C_2$-alkyl, $C_1$-$C_4$-alkylaminocarbonyl-$C_1$-$C_2$-alkyl, benzyl, trimethylsilylmethyl or an ammonium equivalent, a $C_1$-$C_3$-alkylammonium equivalent, a sodium equivalent or a potassium equivalent, or the group

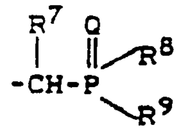

wherein

$R^7$ represents hydrogen, methyl, phenyl, furyl, thienyl or pyridyl,

$R^8$ represents methoxy or ethoxy,

$R^9$ represents methoxy or ethoxy and

Q represents oxygen or sulphur

or

$R^6$ represents the $(-CH_2-)_n-R^{10}$ group,

wherein

n represents the numbers 0, 1 or 2 and

$r^{10}$ represents a heterocyclic radical from the series comprising furyl, tetrahydrofuryl, thienyl, perhydropyranyl, oxazolyl, thiazolyl and dioxolanyl, each of which is optionally substituted by chlorine and/or methyl.

4. R enantiomers of the formula (I) according to Claim 3.

5. Process for the preparation of the α-(5-aryloxynaphthalen-1-yl-oxy)-propionic acid derivatives of the formula (I)

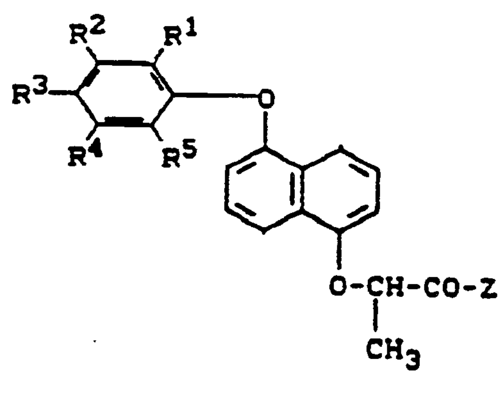

(I)

in which

$R^1$ represents hydrogen, halogen, cyano or trifluoromethyl,
$R^2$ represents hydrogen or halogen,
$R^3$ represents halogen, cyano, trifluoromethyl or trifluoromethoxy,
$R^4$ represents hydrogen or halogen,
$R^5$ represents hydrogen or halogen and Z represents halogen, hydroxyl, amino, alkylamino, alkenylamino, alkinylamino, arylamino, aralkylamino, alkoxycarbonylalkylamino, cyanamido, dialkylamino, dialkenylamino, alkylsulphonylamino, arylsulphonylamino, hydroxylamino, alkoxyamino, hydrazino, alkylsulphonylhydrazino, arylsulphonylhydrazino, alkylthio, arylthio, aralkylthio, alkoxycarbonylalkylthio or the $-O-R^{6s}$ group,
wherein
$R^6$ represents an optionally halogen-substituted radical from the series comprising alkyl, alkenyl, alkinyl, alkoxyalkyl, alkylthioalkyl, alkylsulphinylalkyl, alkylsulphonylalkyl, aryloxyalkyl, trialkylsilylalkyl, arylthioalkyl, aralkoxyalkyl, aralkylthioalkyl, alkoxycarbonylalkyl, alkylaminocarbonylalkyl, aralkyl, azolylalkyl, alkylideneamino or an ammonium equivalent, an alkylammonium equivalent, an alkali metal equivalent or an alkaline earth metal equivalent or the group

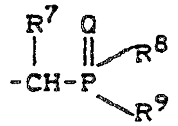

wherein
$R^7$ represents hydrogen, alkyl, aryl, furyl, thienyl or pyridyl,
$R^8$ represents alkyl or alkoxy,
$R^9$ represents alkoxy and
Q represents oxygen or sulphur,
or
$R^6$ represents the $-(CH_2)_n-R^{10}$ group,
wherein
$R^{10}$ represents a heterocyclic radical from the series comprising furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl, each of which is optionally substituted by halogen and/or alkyl, and
n represents the numbers 0, 1 or 2, characterised in that
(a) 5-aryloxy-1-naphthols of the general formula (II)

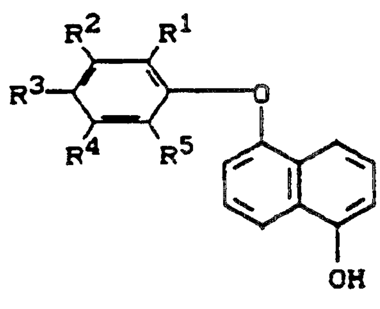 (II)

in which
$R^1$, $R^2$, $R^3$, $R^4$ and $R^5$ have the abovementioned meanings, are reacted with propionic acid derivatives of the general formula (III)

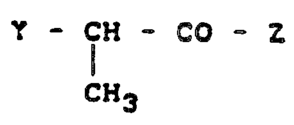 (III)

in which
Z has the abovementioned meaning and
Y represents a nucleophilic leaving group, if appropriate in the presence of an acid acceptor and if appropriate in the presence of a diluent, or

(b) halogeno-benzene derivatives of the general formula (IV)

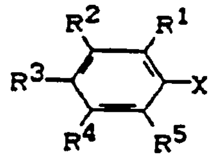

(IV)

in which
$R^1$, $R^2$, $R^3$, $R^4$ and $R^5$ have the abovementioned meanings and
X represents halogen are reacted with α-(5-hydroxy-naphthalen-1-yl-oxy)-propionic acid derivatives of the general formula (V)

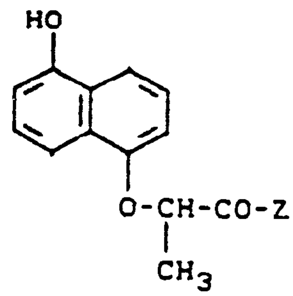

(V)

in which
Z has the abovementioned meaning, if appropriate in the presence of an acid acceptor and if appropriate in the presence of a diluent, or
(c) for the case in which Z represents hydroxyl and the radicals $R^1$ to $R^5$ have the abovementioned meanings, compounds of the general formula (I) in which Z represents methoxy or ethoxy, and the radicals $R^1$ to $R^5$ have the abovementioned meanings, are reacted with an alkali metal hydroxide in the presence of water and if appropriate in the presence of an organic solvent and are then acidified – if appropriate after concentration – using a mineral acid, or
(d) for the case in which Z represents halogen and the radicals $R^1$ to $R^5$ have the abovementioned meanings, compounds of the general formula (I) in which Z represents hydroxyl and the radicals $R^1$ to $R^5$ have the abovementioned meanings, are reacted with a halogenating agent, if appropriate in the presence of a catalyst and if appropriate in the presence of a diluent, or
(e) for the case in which Z, with the exception of halogen, has the abovementioned meaning and the radicals $R^1$ to $R^5$ have the abovementioned meanings, compounds of the general formula (I) in which Z represents halogen and the radicals $R^1$ to $R^5$ have the abovementioned meanings, are reacted with compounds of the general formula (VI)

$$H-Z \quad (VI)$$

in which
Z, with the exception of halogen, has the abovementioned meaning,
if appropriate in the presence of an acid acceptor and if appropriate in the presence of a diluent, or
(f) for the case in which Z represents the $-O-R^6$ group, wherein $R^6$, with the exception of ammonium, alkylammonium, alkali metal and alkaline earth metal, has the abovementioned meaning and the radicals $R^1$ to $R^5$ have the abovementioned meanings, compounds of the general formula (I) in which Z represents hydroxyl and the radicals $R^1$ to $R^5$ have the abovementioned meanings, are reacted with compounds of the general formula (VIII)

$$X^1-R^6 \quad (VII)$$

in which
$R^6$, with the exception of ammonium, alkylammonium, alkali metal and alkaline earth metal, has the abovementioned meanings and
$X^1$ represents halogen,

if appropriate in the presence of an acid acceptor and if appropriate in the presence of a diluent.

6. Herbicidal agents, characterized in that they contain at least one α-(5-aryloxy-naphthalen-1-yl-oxy)-propionic acid derivative of the formula (I) according to Claims 1 to 5.

7. Method for combating weeds, characterized in that α-(5-aryloxy-naphthalen-1-yl-oxy)-propionic acid derivatives of the formula (I) according to Claims 1 to 5 are allowed to act on the weeds and/or their environment.

8. Use of α-(5-aryloxy-naphthalen-1-yl-oxy)-propionic acid derivatives of the formula (I) according to Claims 1 to 5 for combating weeds.

9. Process for the production of herbicidal agents, characterized in that α-(5-aryloxy-naphthalen-1-yl-oxy)-propionic acid derivatives of the formula (I) according to Claims 1 to 5 are mixed with extenders and/or surface-active substances.

10. 5-Aryloxy-1-naphthols of the formula (II)

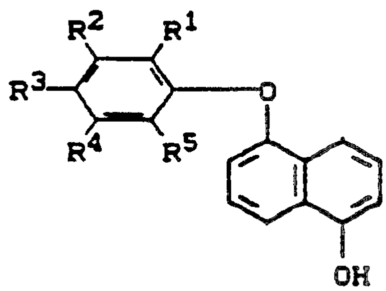

(II)

in which

$R^1$ represents hydrogen, halogen, cyano or trifluoromethyl,
$R^2$ represents hydrogen or halogen
$R^3$ represents halogen, cyano, trifluoromethyl or trifluoromethoxy,
$R^4$ represents hydrogen or halogen, and
$R^5$ represents hydrogen or halogen.

## Revendications

1. Dérivés d'acides α-(5-aryloxy-naphtalène-1-yl-oxy)-propioniques de formule I

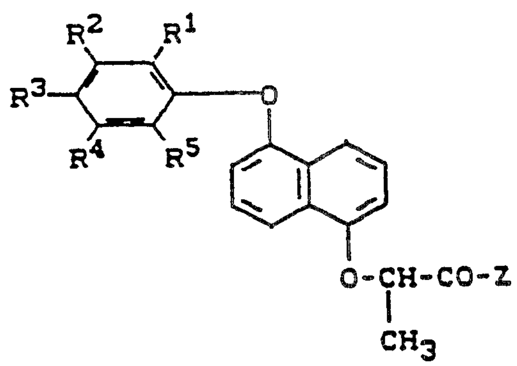

(I)

dans laquelle

$R^1$ représente l'hydrogène, un groupe cyano ou trifluorométhyle,
$R^2$ représente l'hydrogène ou un halogène,
$R^3$ représente un halogène, un groupe cyano, trifluorométhyle ou trifluorométhoxy,
$R^4$ représente l'hydrogène ou un halogène,
$R^5$ représente l'hydrogène ou un halogène, et
Z représente un halogène, un groupe hydroxy, amino, alkylamino, alcénylamino, alcynylamino, arylamino, aralkylamino, alcoxycarbonylalkylamino, cyanamino, dialkylamino, dialcénylamino, alkylsulfonylamino, arylsulfonylamino, hydroxyamino, alcoxyamino, hydrazino, alkylsulfonylhydrazino, arylsulfonylhydrazino, alkylthio, arylthio, aralkylthio, alcoxycarbonylalkylthio ou le groupement $-O-R^6$ dans lequel

$R^6$ représente un radical éventuellement substitué par des halogènes, choisi parmi les radicaux alkyle, alcényle, alcynyle, alcoxyalkyle, alkylthioalkyle, alkylsulfinylalkyle, alkylsulfonylalkyle, aryloxyalkyle, trialkylsilylalkyle, arylthioalkyle, aralcoxyalkyle, aralkylthioalkyle, alcoxycarbonylalkyle, alkylaminocarbonylalkyle, aralkyle, azolylalkyle, alkylidèneamino ou un équivalent d'ammonium, d'alkylammonium, de métal alcalin ou alcalino-terreux, ou bien encore le groupement

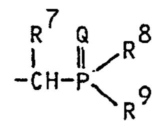

dans lequel

$R^7$ représente l'hydrogène, un groupe alkyle, aryle, furyle, thiényle ou pyridyle,

$R^8$ représente un groupe alkyle ou alcoxy,

$R^9$ représente un groupe alcoxy, et

Q représente l'oxygène ou le soufre, ou bien

$R^6$ représente le groupement $-(CH_2)_n-R^{10}$ dans lequel

$R^{10}$ représente un radical hétérocyclique éventuellement substitué par des halogènes et/ou des groupes alkyle et choisi parmi les radicaux furyle, tétrahydrofuryle, oxotétrahydrofuryle, thiényle, tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle, et

n est égal a 0, 1 ou 2.

2. Dérivés d'acides α-5-(aryloxy-naphtalène-1-yl-oxy)-propioniques de formule I de la revendication 1, dans laquelle

$R^1$ représente l'hydrogène, le fluor, le chlore, le brome, un groupe cyano, ou trifluorométhyle,

$R^2$ représente l'hydrogène, le fluor ou le chlore,

$R^3$ représente le fluor, le chlore, le brome, un groupe cyano, trifluorométhyle ou trifluorométhoxy,

$R^4$ représente l'hydrogène, le fluor ou le chlore,

$R^5$ représente l'hydrogéne, le fluor, le chlore ou le brome, et

Z représente le chlore, un groupe hydroxy, amino, alkylamino en $C_1$-$C_6$, alcénylamino en $C_3$-$C_4$, alcynylamino en $C_3$-$C_4$, phénylamino, benzylamino, (alcoxy en $C_1$-$C_4$)-carbonyl-alkylamino en $C_1$-$C_2$, cyanamino, di-(alkyle en $C_1$-$C_4$)-amino, di-(alcényle en $C_3$-$C_4$)-amino, alkylsulfonylamino en $C_1$-$C_4$, phénylsulfonylamino, tolylsulfonylamino, hydroxyamino, alcoxyamino en $C_1$-$C_6$, hydrazino, (alkyle en $C_1$-$C_4$)-sulfonylhydrazino, phénylsulfonylhydrazino, tolylsulfonylhydrazino, alkylthio en $C_1$-$C_4$, phénylthio, benzylthio, (alcoxy en $C_1$-$C_4$)-carbonyl-alkylthio en $C_1$-$C_2$ ou le groupement $-O-R^6$ dans lequel

$R^6$ représente un radical éventuellement substitué par le fluor et/ou le chlore et choisi parmi les radicaux alkyle en $C_1$-$C_6$, alcényle en $C_3$-$C_4$, alcynyle en $C_3$-$C_4$, (alcoxy en $C_1$-$C_4$)-alkyle en $C_1$-$C_4$, (alkylthio en $C_1$-$C_4$)-alkyle en $C_1$-$C_4$, (alkyle en $C_1$-$C_4$)-sulfinyl-alkyle en $C_1$-$C_4$, (alkyle en $C_1$-$C_4$)-sulfonyl-alkyle en $C_1$-$C_4$, phénoxy-alkyle en $C_1$-$C_3$, triméthylsilylméthyle, phényl-thio-alkyle en $C_1$-$C_3$, benzyloxy-alkyle en $C_1$-$C_3$, benzylthioalkyle en $C_1$-$C_3$, (alcoxy en $C_1$-$C_4$)-carbonyl-alkyle en $C_1$-$C_2$, (alkylamino en $C_1$-$C_4$)-carbonyl-alkyle en $C_1$-$C_2$, benzyle, pyrazolyl-alkyle en $C_1$-$C_4$, alkylidène-amino en $C_2$-$C_4$ ou un equivalent d'ammonium, d'alkylammonium en $C_1$-$C_4$, de sodium, de potassium ou de calcium, ou bien le groupement

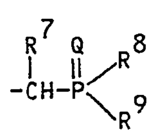

dans lequel

$R^7$ représente l'hydrogène, un groupe alkyle en $C_1$-$C_4$, phényle, furyle, thiényle ou pyridyle,

$R^8$ représente un groupe alkyle en $C_1$-$C_4$ ou alcoxy en $C_1$-$C_4$,

$R^9$ représente un groupe alcoxy en $C_1$-$C_4$ et

Q représente l'oxygène ou le soufre, ou bien

$R^6$ représente le groupement $-(CH_2)_n-R^{10}$ dans lequel

n est égal à 0, 1 ou 2,

$R^{10}$ représente un radical hétérocyclique éventuellement substitué par le fluor, le chlore, le brome et/ou des groupes alkyle en $C_1$-$C_4$ et choisi parmi les radicaux furyle, tétrahydrofuryle, oxotétrahydrofuryle, thiényle,

tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle.

3. Dérivés d'acides α-(5-aryloxynaphtalène-1-yl-oxy)-propioniques de formule I de la revendication 1, dans laquelle

$R^1$ représente l'hydrogène, un groupe cyano, le fluor ou le chlore,

$R^2$ représente l'hydrogène, le fluor ou le chlore,

$R^3$ représente un groupe trifluorométhyle,

$R^4$ représente l'hydrogène, le fluor ou le chlore,

$R^5$ représente l'hydrogène, le fluor ou le chlore, et

Z représente le chlore, un groupe hydroxy, amino, alkylamino en $C_1$-$C_4$, phénylamino, (alcoxy en $C_1$-$C_4$)-carbonyl-alkylamino en $C_1$-$C_2$, di-(alkyle en $C_1$-$C_3$)-amino, alkylsulfonylamino en $C_1$-$C_4$, phénylsulfonylamino, hydroxyamino, cyanamino, alcoxyamino en $C_1$-$C_4$, hydrazino, (alkyle en $C_1$-$C_4$)-sulfonylhydrazino, phénylsulfonylhydrazino, alkylthio en $C_1$-$C_4$ ou (alcoxy en $C_1$-$C_4$)-carbonylalkylthio en $C_1$-$C_2$, ou bien le groupement –O-$R^6$ dans lequel

$R^6$ représente un groupe alkyle en $C_1$-$C_4$, (alcoxy en $C_1$-$C_2$)-alkyle en $C_1$-$C_2$, (alkylthio en $C_1$-$C_2$)-alkyle en $C_1$-$C_2$, (alkylsulfinyle en $C_1$-$C_2$)-alkyle en $C_1$-$C_2$, (alkylsulfonyle en $C_1$-$C_2$)-alkyle en $C_1$-$C_2$, benzyloxy-alkyle en $C_1$-$C_3$, benzylthio-alkyle en $C_1$-$C_3$, (alcoxy en $C_1$-$C_4$)-carbonyl-alkyle en $C_1$-$C_2$, (alkylamino en $C_1$-$C_4$)-carbonyl-alkyle en $C_1$-$C_2$, benzyle, triméthylsilylméthyle ou un équivalent d'ammonium, d'alkylammonium en $C_1$-$C_3$, de sodium ou de potassium, ou bien le groupement

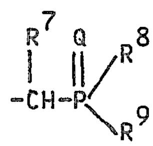

dans lequel,

$R^7$ représente l'hydrogène, un groupe méthyle, phényle, furyle, thiényle ou pyridyle,

$R^8$ représente un groupe méthoxy ou éthoxy,

$R^9$ représente un groupe méthoxy ou éthoxy, et

Q représente l'oxygène ou le soufre,

ou bien

$R^6$ représente le groupement $(-CH_2-)_n-R^{10}$ dans lequel

n est égal à 0, 1 ou 2, et

$R^{10}$ représente un radical hétérocyclique éventuellement substitué par le chlore et/ou des groupes méthyle et choisi parmi les groupes furyle, tétrahydrofuryle, thiényle, perhydropyrannyle, oxazolyle, thiazolyle et dioxolannyle.

4. Enantiomères R de formule I de la revendication 3.

5. Procédé de préparation des dérivés d'acides α-(5-aryl-oxy-naphtalène-1-yl-oxy)-propioniques de formule I

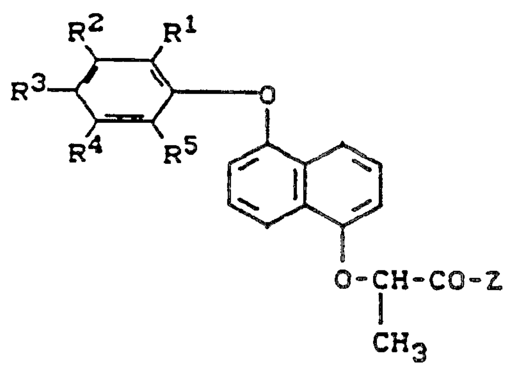

(I)

dans laquelle

$R^1$ représente l'hydrogène, un halogène, un groupe cyano ou trifluorométhyle,

$R^2$ représente l'hydrogène ou un halogène,

$R^3$ représente un halogène, un groupe cyano, trifluorométhyle ou trifluorométhoxy,

$R^4$ représente l'hydrogène ou un halogéne,

$R^5$ représente l'hydrogène ou un halogène, et

Z représente un halogène, un groupe hydroxy, amino, alkylamino, alcénylamino, alcynylamino, arylamino, aralkylamino, alcoxycarbonylalkylamino, cyanamino, dialkylamino, dialcénylamino, alkylsulfonylamino, arylsulfonylamino, hydroxyamino, alcoxyamino, hydrazino, alkylsulfonylhydrazino, arylsulfonylhydrazino, alkylthio, arylthio, aralkylthio, alcoxycarbonylalkylthio ou le groupement $-O-R^6$ dans lequel

$R^6$ représente un radical éventuellement substitué par des halogènes et choisi parmi les radicaux alkyle, alcényle, alcynyle, alcoxyalkyle, alkylthioalkyle, alkylsulfinylalkyle, alkylsulfonylalkyle, aryloxyalkyle, trialkylsilylalkyle, arylthioalkyle, aralcoxyalkyle, aralkylthioalkyle, alcoxycarbonylalkyle, alkylaminocarbonylalkyle, aralkyle, azolylalkyle, alkylidène-amino ou un équivalent d'ammonium, d'alkylammonium, de métal alcalin ou alcalino-terreux, ou bien le groupement

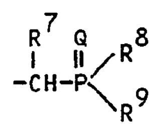

dans lequel

$R^7$ représente l'hydrogène, un groupe alkyle, aryle, furyle, thiényle ou pyridyle,

$R^8$ représente un groupe alkyle ou alcoxy,

$R^9$ représente un groupe alcoxy, et

Q représente l'oxygène ou le soufre,

ou bien

$R^6$ représente le groupement $-(CH_2)_n-R^{10}$ dans lequel

$R^{10}$ représente un radical hétérocyclique éventuellement substitué par des halogènes et/ou des groupes alkyle et choisi parmi les radicaux furyle, tétrahydrofuryle, oxotétrahydrofuryle, thiényle, tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle, et

n est égal à 0, 1 ou 2,

caractérisé en ce que :

a) on fait réagir des 5-aryloxy-1-naphtols de formule générale II

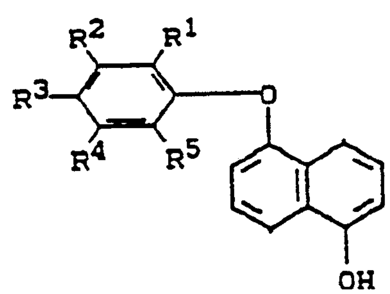

(II)

dans laquelle $R^1$, $R^2$, $R^3$, $R^4$ et $R^5$ ont les significations indiquées ci-dessus, avec des dérivés de l'acide propionique de formule générale III

$$Y - \underset{\displaystyle CH_3}{\underset{|}{CH}} - CO - Z \qquad (III)$$

dans laquelle

Z a les significations indiquées ci-dessus, et

Y représente un groupe éliminable nucléophile, éventuellement en présence d'un accepteur d'acide et éventuellement en présence d'un diluant, ou bien

b) on fait réagir des dérivés d'halogénobenzènes de formule générale IV

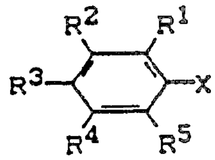

(IV)

dans laquelle
$R^1$, $R^2$, $R^3$, $R^4$ et $R^5$ ont les significations indiquées ci-dessus, et
X représente un halogène, avec des dérivés de l'acide α-(5-hydroxy-naphtalène-1-yl-oxy)-propionique de formule générale V

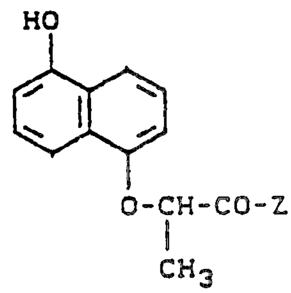

(V)

dans laquelle
Z a les significations indiquées ci-dessus, éventuellement en présence d'un accepteur d'acide et éventuellement en présence d'un diluant, ou bien
c) dans le cas où Z représente un groupe hydroxy et les symboles $R^1$ à $R^5$ ont les significations indiquées ci-dessus, on fait réagir des composés répondant à la formule générale I dans laquelle Z représente un groupe méthoxy ou éthoxy et les symboles $R^1$ à $R^5$ ont les significations indiquées ci-dessus, avec un hydroxyde alcalin en présence d'eau et le cas échéant en présence d'un solvant organique puis – éventuellement après concentration – on acidifie par un acide minéral, ou bien
d) dans le cas où Z représente un halogène et les symboles $R^1$ à $R^5$ ont les significations indiquées ci-dessus, on fait réagir des composés de formule générale I dans laquelle Z représente un groupe hydroxy et les symboles $R^1$ à $R^5$ ont les significations indiquées ci-dessus, avec un agent halogénant, éventuellement en présence d'un catalyseur et éventuellement en présence d'un diluant, ou bien
e) dans le cas où Z a les significations indiquées ci-dessus, à l'exception d'un halogéne, et les symboles $R^1$ à $R^5$ ont les significations indiquées ci-dessus, on fait réagir des composés de formule générale I dans laquelle Z représente un halogène et $R^1$ à $R^5$ ont les significations indiquées ci-dessus, avec des composés de formule générale VI

$$H-Z \quad (VI)$$

dans laquelle Z a les significations indiquées ci-dessus, à l'exception d'un halogène, éventuellement en présence d'un accepteur d'acide et éventuellement en présence d'un diluant, ou bien
f) dans le cas où Z représente le groupement $-O-R^6$ dans lequel $R^6$ a les significations indiquées ci-dessus, à l'exception de l'ammonium, d'un alkylammonium, d'un métal alcalin ou alcalino-terreux et les symboles $R^1$ à $R^5$ ont les significations indiquées ci-dessus, on fait réagir des composés de formule générale I dans laquelle Z représente un groupe hydroxy et les symboles $R^1$ à $R^5$ ont les significations indiquées ci-dessus, avec des composés de formule générale VII

$$X^1-R^6 \quad (VII)$$

dans laquelle
$R^6$ a les significations indiquées ci-dessus, à l'exception de l'ammonium, d'un alkylammonium, d'un métal alcalin ou alcalino-terreux, et
$X^1$ représente un halogène, éventuellement en présence d'un accepteur d'acide et éventuellement en présence d'un diluant.

6. Produits herbicides, caractérisé en ce qu'ils contiennent au moins un dérivé d'acide α-(5-aryloxy-naphtalène-1-yl-oxy)-propionique de formule I selon les revendications 1 à 5.

7. Procédé pour combattre les végétaux adventices, caractérisé en ce que l'on fait agir sur ces végétaux et/ou leur habitat des dérivés d'acides α-(5-aryloxy-naphtalène-1-yl-oxy)-propioniques de formule I selon les revendications 1 à 5.

8. Utilisation des dérivés d'acides α-(5-aryloxynaphtalène-1-yl-oxy)-propioniques de formule I selon les revendications 1 à 5 pour la lutte contre les végétaux adventices.

9. Procédé de préparation de produits herbicides, caractérisé en ce que l'on mélange des dérivés d'acides α-(5-aryloxynaphtalène-1-yl-oxy)-propioniques de formule I selon les revendications 1 à 5 avec des diluants et/ou des agents tensioactifs.

10. 5-aryloxy-1-naphtols de formule II

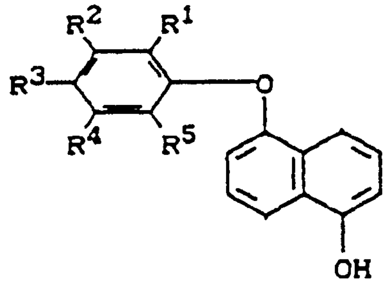

(II)

dans laquelle

$R^1$ représente l'hydrogène, un halogène, un groupe cyano ou trifluorométhyle,
$R^2$ représente l'hydrogéne ou un halogène,
$R^3$ représente un halogène, un groupe cyano, trifluorométhyle ou trifluorométhoxy,
$R^4$ représente l'hydrogène ou un halogène,
$R^5$ représente l'hydrogène ou un halogène.